(19) Europäisches Patentamt
European Patent Office
Office européen des brevets

(11) EP 0 757 669 B1

(12) **EUROPÄISCHE PATENTSCHRIFT**

(45) Veröffentlichungstag und Bekanntmachung des Hinweises auf die Patenterteilung:
**18.11.1998 Patentblatt 1998/47**

(21) Anmeldenummer: **94915531.1**

(22) Anmeldetag: **25.04.1994**

(51) Int Cl.[6]: **C07C 219/24**, C07D 295/08, A61K 31/22, A61K 31/24, A61K 31/445, A61K 31/495, A61K 31/535

(86) Internationale Anmeldenummer:
**PCT/EP94/01276**

(87) Internationale Veröffentlichungsnummer:
**WO 95/29148 (02.11.1995 Gazette 1995/47)**

(54) **O-ACYL-4-PHENYL-CYCLOHEXANOLE, DEREN SALZE, DIESE VERBINDUNGEN ENTHALTENDE ARZNEIMITTEL UND DEREN VERWENDUNG SOWIE VERFAHREN ZU IHRER HERSTELLUNG**

O-ACYL-4-PHENYL-CYCLOHEXANOLS, THEIR SALTS, MEDICAMENTS CONTAINING SUCH COMPOUNDS AND THEIR USE, AS WELL AS A METHOD OF PREPARING THEM

O-ACYL-4-PHENYL-CYCLOHEXANOLS, LEURS SELS, MEDICAMENTS CONTENANT CES COMPOSES ET LEUR UTILISATION, AINSI QUE LEUR PROCEDE DE FABRICATION

(84) Benannte Vertragsstaaten:
**AT BE CH DE DK ES FR GB GR IE IT LI LU MC NL PT SE**

(43) Veröffentlichungstag der Anmeldung:
**12.02.1997 Patentblatt 1997/07**

(73) Patentinhaber: **Boehringer Ingelheim Pharma KG**
**55216 Ingelheim am Rhein (DE)**

(72) Erfinder:
- **WOITUN, Eberhard**
  **D-88400 Biberach (DE)**
- **MAIER, Roland**
  **D-88400 Biberach (DE)**
- **MÜLLER, Peter**
  **D-88441 Mittelbiberach (DE)**
- **HURNAUS, Rudolf**
  **D-88400 Biberach (DE)**
- **MARK, Michael**
  **D-88400 Biberach (DE)**
- **EISELE, Bernhard**
  **D-88400 Biberach (DE)**
- **BUDZINSKI, Ralph-Michael**
  **D-88400 Biberach (DE)**
- **HALLERMAYER, Gerhard**
  **D-88437 Maselheim-Sulmingen (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 464 465** **DE-A- 4 239 150**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 757 669 B1

Printed by Jouve, 75001 PARIS (FR)

**Beschreibung**

Die vorliegende Erfindung betrifft O-Acyl-4-phenyl-cycloalkanole, ihre Salze mit physiologisch verträglichen organischen und anorganischen Säuren, Verfahren zur Herstellung dieser Verbindungen und diese enthaltende Arzneimittel und deren Verwendung.

Die erfindungsgemäßen Verbindungen stellen Inhibitoren der Cholesterolbiosynthese dar, insbesondere Inhibitoren des Enzyms 2,3-Epoxisqualen-Lanosterol-Cyclase, eines Schlüsselenzyms der Cholesterolbiosynthese. Die erfindungsgemäßen Verbindungen sind geeignet zur Behandlung und Prophylaxe von Hyperlipidämien, Hypercholesterolämien und der Atherosklerose. Weitere mögliche Anwendungsgebiete ergeben sich für die Behandlung von hyperproliferativen Haut- und Gefäßerkrankungen, Tumoren, Gallensteinleiden sowie von Mykosen.

Verbindungen, die in die Cholesterolbiosynthese eingreifen, sind für die Behandlung einer Reihe von Krankheitsbildern von Bedeutung. Hier sind vor allem Hypercholesterolämien und Hyperlipidämien zu nennen, die Risikofaktoren für das Entstehen atherosklerotischer Gefäßveränderungen und ihrer Folgeerkrankungen wie beispielsweise koronare Herzkrankheit, cerebrale Ischämie, Claudicatio intermittens und Gangrän darstellen.

Die Bedeutung überhöhter Serum-Cholesterol-Spiegel als Hauptrisikofaktor für das Entstehen atherosklerotischer Gefäßveränderungen wird allgemein anerkannt. Umfangreiche klinische Studien haben zu der Erkenntnis geführt, daß durch Erniedrigung des Serumcholesterols das Risiko für koronare Herzerkrankungen verkleinert werden kann (Current Opinion in Lipidology 2(4), 234 [1991]). Da der größte Teil des Cholesterols im Organismus selbst synthetisiert und nur ein geringer Teil mit der Nahrung aufgenommen wird, stellt die Hemmung der Biosynthese einen besonders attraktiven Weg dar, den erhöhten Cholesterolspiegel zu senken.

Daneben kommen als weitere mögliche Anwendungsgebiete von Cholesterolbiosynthesehemmern die Behandlung hyperproliferativer Haut- und Gefäßerkrankungen sowie von Tumorerkrankungen, die Behandlung und Prophylaxe von Gallensteinleiden sowie der Einsatz bei Mykosen in Betracht. Im letzteren Fall handelt es sich um einen Eingriff in die Ergosterolbiosynthese in Pilzorganismen, welche weitgehend analog der Cholesterolbiosynthese in Säugerzellen verläuft.

Die Cholesterol- bzw. die Ergosterolbiosynthese verläuft, ausgehend von Essigsäure, über eine größere Zahl von Reaktionsschritten. Dieser Vielstufenprozeß bietet eine Reihe von Eingriffsmöglichkeiten, von denen als Beispiele genannt seien:

Für die Inhibition des Enzyms 3-Hydroxy-3-methylglutaryl-Coenzym A (HMG-CoA)-Synthase werden β-Lactone und β-Lactame mit potentieller antihypercholesterolämischer Wirkung erwähnt (siehe J. Antibiotics 40, 1356 [1987], US-A-4,751,237, EP-A-0 462 667, US-A-4,983,597).

Inhibitoren des Enzyms HMG-CoA-Reduktase stellen 3,5-Dihydroxycarbonsäuren vom Statintyp und deren δ-Lactone dar, deren Vertreter Lovastatin, Simvastatin und Pravastatin in der Therapie von Hypercholesterolämien Verwendung finden.

Weitere mögliche Anwendungsgebiete dieser Verbindungen sind Pilzinfektionen (US-A-4,375,475, EP-A-0 113 881, US-A-5,106,992), Hauterkrankungen (EP-A-0 369 263) sowie Gallensteinleiden und Tumorerkrankungen (US-A-5,106,992; Lancet 339, 1154-1156 [1992]). Eine weitere Therapiemöglichkeit stellt die Hemmung der Proliferation glatter Muskelzellen durch Lovastatin dar (Cardiovasc. Drugs. Ther. 5, Suppl. 3, 354 [1991]).

Inhibitoren des Enzyms Squalen-Synthetase sind z.B. Isoprenoid-(phosphinylmethyl)phosphonate, deren Eignung zur Behandlung von Hypercholesterolämien, Gallensteinleiden und Tumorerkrankungen beschrieben ist in EP-A-0 409 181 sowie J. Med. Chemistry 34, 1912 [1991], ferner die Squalestatine mit cholesterolsenkender und antimykotischer Wirkung (J. Antibotics 45, 639-647 [1992] und J. Biol. Chemistry 267, 11705-11708 [1992].

Als Inhibitoren des Enzyms Squalen-Epoxidase sind bekannt Allylamine wie Naftifin und Terbinafin, die als Mittel gegen Pilzerkrankungen Eingang in die Therapie gefunden haben, sowie das Allylamin NB-598 mit antihypercholesterolämischer Wirkung (J. Biol. Chemistry 265, 18075-18078, [1990]) und Fluorsqualen-Derivate mit hypocholesterolämischer Wirkung (US-A-5,011,859). Des weiteren sind Piperidine und Azadecaline mit potentieller hypocholesterolämischer und/oder antifungaler Wirkung beschrieben, deren Wirkmechanismus nicht eindeutig geklärt ist und welche Squalenepoxidase- und/oder 2,3-Epoxisqualen-Lanosterol-Cyclase-Inhibitoren darstellen (EP-A-0 420 116, EP-A-0 468 434, US-A-5,084,461 und EP-A-0 468 457).

Beispiele für Inhibitoren des Enzyms 2,3-Epoxisqualen-Lanosterol-Cyclase sind Diphenylderivate (EP-A-0 464 465), Aminoalkoxybenzyl-Derivate (EP-A-0 410 359) sowie Piperidin-Derivate (J. Org. Chem. 57, 2794-2803, [1992]), die eine anti-fungale Wirkung besitzen. Des weiteren wird dieses Enzym in Säugetierzellen durch Decaline, Azadecaline und Indanderivate (WO 89/08450, J. Biol. Chemistry 254, 11258-11263 [1981], Biochem. Pharmacology 37, 1955-1964 [1988] und J 64 003 144), ferner durch 2-Aza-2,3-dihydrosqualen und 2,3-Epiminosqualen (Biochem. Pharmacology 34, 2765-2777 [1985]), durch Squalenoxid-Epoxid-Enolether (J. Chem. Soc. Perkin Trans. I, 1988, 461) und 29-Methyliden-2,3-oxidosqualen (J. Amer. Chem. Soc. 113, 9673-9674 [1991]) inhibiert.

Schließlich sind als Inhibitoren des Enzyms Lanosterol-14α-Demethylase noch Steroidderivate mit potentieller antihyperlipämischer Wirkung zu nennen, die gleichzeitig das Enzym HMG-CoA-Reduktase beeinflussen (US-A-5,041,432, J. Biol. Chemistry 266, 20070-20078 [1991], US-A-5,034,548). Außerdem wird dieses Enzym durch die Antimykotika vom Azol-Typ inhibiert, welche N-substituierte Imidazole und Triazole darstellen. Zu dieser Klasse gehören beispielsweise die auf dem Markt befindlichen Antimykotika Ketoconazol und Fluconazol.

Die Verbindungen der nachfolgenden allgemeinen Formel I sind neu. Es wurde überraschenderweise gefunden, daß sie sehr wirksame Inhibitoren des Enzyms 2,3-Epoxisqualen-Lanosterol-Cyclase (Internationale Klassifizierung: EC5.4.99.7) darstellen.

Das Enzym 2,3-Epoxisqualen-Lanosterol-Cyclase katalysiert einen Schlüsselschritt der Cholesterol- bzw. Ergosterol-Biosynthese, nämlich die Umwandlung des 2,3-Epoxisqualens in das Lanosterol, die erste Verbindung mit Steroidstruktur in der Biosynthesekaskade. Inhibitoren dieses Enzyms lassen gegenüber Inhibitoren früherer Biosyntheseschritte, wie beispielsweise der HMG-CoA-Synthese und der HMG-CoA-Reduktion, den Vorteil der höheren Selektivität erwarten, da die Inhibierung dieser frühen Biosyntheseschritte zur Abnahme biosynthetisch gebildeter Mevalonsäure führt und dadurch auch die Biosynthese der mevalonsäureabhängigen Substanzen Dolichol, Ubichinon und Isopentenyl-t-RNA negativ beeinflussen kann (vgl. J. Biol. Chemistry 265, 18075-18078 [1990]).

Bei Inhibierung von Biosyntheseschritten nach der Umwandlung von 2,3-Epoxisqualen in Lanosterol besteht die Gefahr der Anhäufung von Intermediärprodukten mit Steroidstruktur im Organismus und der Auslösung dadurch bedingter toxischer Effekte. Dies ist beispielsweise für Triparanol, einem Desmosterol-Reduktase-Inhibitor, beschrieben. Diese Substanz mußte wegen Bildung von Katarakten, Ichthyosis und Alopecie vom Markt genommen werden (zitiert in J. Biol. Chemistry 265, 18075-18078 [1990]).

Wie bereits eingangs dargelegt sind Inhibitoren der 2,3-Epoxisqualen-Lanosterol-Cyclase vereinzelt in der Literatur beschrieben. Die Strukturen dieser Verbindungen sind jedoch völlig verschieden von der Struktur der erfindungsgemäßen Verbindungen der nachstehend genannten allgemeinen Formel I.

Die Erfindung betrifft die Bereitstellung von antihypercholesterolämischen Substanzen, die zur Behandlung und Prophylaxe der Atherosklerose geeignet sind und, im Vergleich zu bekannten Wirkstoffen, durch eine bessere antihypercholesterolämische Wirkung bei erhöhter Selektivität und damit erhöhter Sicherheit ausgezeichnet sind. Da die erfindungsgemäßen Verbindungen auf Grund ihrer hohen Wirksamkeit als Inhibitoren des Enzyms 2,3-Epoxisqualen-Lanosterol-Cyclase auch die Ergosterol-Biosynthese im Pilzorganismus inhibieren können, sind sie auch zur Behandlung von Mykosen geeignet.

Die vorliegende Erfindung hat neue O-Acyl-4-phenyl-cyclohexanole der allgemeinen Formel I zum Gegenstand,

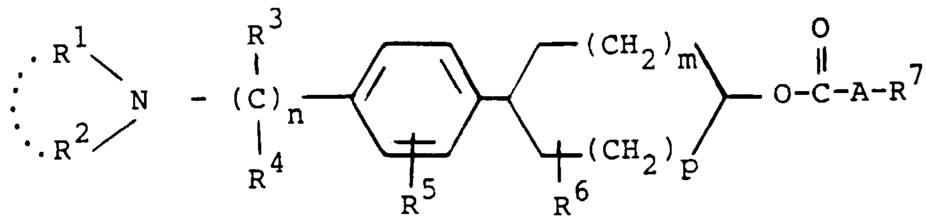

, (I)

in der

n, m und p jeweils die Zahl 1,

$R^1$ und $R^2$, die gleich oder verschieden sein können, ein Wasserstoffatom, eine geradkettige oder verzweigte Alkylgruppe mit 1 bis 6 Kohlenstoffatomen, eine geradkettige oder verzweigte Alkenyl- oder Alkinylgruppe mit jeweils 3 bis 6 Kohlenstoffatomen, wobei deren Doppel- und Dreifachbindungen von der Stickstoff-Kohlenstoff-Bindung isoliert sind und wobei die vorstehend erwähnte Alkyl-, Alkenyl- und Alkinylgruppe durch eine Amino-, Hydroxy-, Alkoxy-, Alkylcarbonyloxy-, Alkylcarbonylamino-, Carboxyl-, Alkoxycarbonyl-, Aminocarbonyl- oder Cyanogruppe substituiert sein kann, wobei die vorstehend erwähnte Amino-, Hydroxy-, Alkoxy-, Alkylcarbonyloxy- und Alkylcarbonylaminogruppe nicht an ein ungesättigtes Kohlenstoffatom und nicht an das Kohlenstoffatom in Position 1 gebunden sein können, oder

$R^1$ und $R^2$ zusammen mit dem dazwischenliegenden Stickstoffatom ein 5- bis 7-gliedriger gesättigter monocyclischer heterocyclischer Ring, wobei in einem so gebildeten 6-gliedrigen gesättigten monocyclischen heterocyclischen Ring eine Methylengruppe in 4-Stellung durch ein Sauerstoff- oder Schwefelatom oder durch eine gegebenenfalls durch eine Alkylgruppe substituierte Iminogruppe ersetzt sein kann,

$R^3$ bis $R^6$, die gleich oder verschieden sein können, jeweils ein Wasserstoffatom oder eine Methylgruppe,

$R^7$ ein Wasserstoffatom, eine Cycloalkylgruppe mit 3 bis 7 Kohlenstoffatomen, eine gegebenenfalls durch ein Fluor-, Chlor- oder Bromatom, durch eine Hydroxy-, Alkyl-, Alkoxy-, Phenylalkoxy-, Phenyl-, Nitro-, Amino-, Alkylamino-, Dialkylamino-, Alkylcarbonylamino-, Cyano-, Carboxy-, Alkoxycarbonyl-, Aminocarbonyl-, Alkyl aminocarbonyl - , Dialkylaminocarbonyl-, Trifluormethyl-, Alkylcarbonyloxy-, Aminosulfonyl-, Alkylaminosulfonyl- oder Dialkylaminosulfonylgruppe mono- oder disubstituierte Phenylgruppe, wobei die Substituenten gleich oder verschieden sein können und wobei zwei benachbarte Wasserstoffatome in einer Phenylgruppe durch eine Methylendioxy- oder 1,2-Ethylendioxygruppe ersetzt sein können, eine durch zwei Chlor- oder Bromatome und eine Aminogruppe substituierte Phenylgruppe, eine Naphthyl- oder Tetrahydronaphthylgruppe, eine durch ein Chlor- oder Bromatom oder durch ein oder zwei Alkylgruppen substituierte Thienyl-, Furyl- oder Pyridylgruppe, und

A eine Bindung, eine geradkettige oder verzweigte Alkylengruppe mit 1 bis 10 Kohlenstoffatomen oder eine Alkenylen- oder Alkinylengruppe mit 2 bis 10 Kohlenstoffatomen bedeuten,

wobei alle vorstehend erwähnten Alkyl- und Alkoxyteile, sofern nichts anderes erwähnt wurde, 1 bis 3 Kohlenstoffatome enthalten können, deren Enantiomere, Diastereomere und geometrischen Isomere sowie deren Salze, insbesondere für die pharmazeutische Anwendung deren physiologisch verträgliche Salze mit anorganischen oder organischen Säuren.

Bevorzugt sind die Verbindungen der allgemeinen Formel I,
in der

n, m und p jeweils die Zahl 1,

$R^1$ ein Wasserstoffatom, eine geradkettige oder verzweigte Alkylgruppe mit 1 bis 4 Kohlenstoffatome, die durch eine Aminocarbonylgruppe oder in 2-, 3- oder 4-Stellung durch eine Hydroxy- oder Alkoxygruppe substituiert sein kann, oder eine 2-Propenylengruppe und

$R^2$ ein Wasserstoffatom, eine Alkylgruppe mit 1 bis 4 Kohlenstoffatomen oder eine 2-Propenylengruppe, oder

$R^1$ und $R^2$ zusammen mit dem dazwischenliegenden Stickstoffatom ein 5- oder 6-gliedriger gesättigter monocyclischer heterocyclischer Ring, wobei in einem so gebildeten 6-gliedrigen gesättigten monocyclischen heterocyclischen Ring eine Methylengruppe in 4-Stellung durch ein Sauerstoffatom oder durch eine gegebenenfalls durch eine Alkylgruppe substituierte Iminogruppe ersetzt sein kann,

$R^3$ bis $R^6$ jeweils ein Wasserstoffatom,

$R^7$ ein Wasserstoffatom, eine Cycloalkylgruppe mit 3 bis 6 Kohlenstoffatomen, eine gegebenenfalls in 4-Stellung durch ein Fluor-, Chlor- oder Bromatom, durch eine Alkyl-, Alkoxy-, Phenyl-, Nitro-, oder Trifluormethylgruppe monosubstituierte Phenylgruppe, eine durch zwei Chloratome, ein Chloratom und eine Alkyl- oder Aminogruppe oder durch zwei Alkoxygruppen disubstituierte Phenylgruppe, eine durch zwei Chloratome und eine Aminogruppe trisubstituierte Phenylgruppe, eine 3,4-Methylendioxyphenylgruppe, eine Naphthyl- oder Tetrahydronaphthylgruppe, eine 2-Furylgruppe oder eine gegebenenfalls in 5-Stellung durch ein Chloratom substituierte 2-Thienylgruppe oder eine 3-Pyridylgruppe,

A eine Bindung, eine geradkettige oder verzweigte Alkylengruppe mit 1 bis 6 Kohlenstoffatomen oder eine Alkenylengruppe mit 2 bis 5 Kohlenstoffatomen bedeuten,

wobei alle vorstehend erwähnten Alkyl- und Alkoxyteile, sofern nichts anderes erwähnt wurde, 1 bis 3 Kohlenstoffatome enthalten können, deren Enantiomere, Diastereomere und geometrischen Isomere sowie deren Salze, insbesondere für die pharmazeutische Anwendung deren physiologisch verträgliche Salze mit anorganischen oder organischen Säuren.

Besonders bevorzugt sind die Verbindungen der allgemeinen Formel I,
in der

n, m und p jeweils die Zahl 1,

$R^1$ ein Wasserstoffatom, eine geradkettige oder verzweigte Alkylgruppe mit 1 bis 4 Kohlenstoffatome, die durch eine Aminocarbonylgruppe oder in 2-, 3- oder 4-Stellung durch eine Hydroxy- oder Alkoxygruppe substituiert sein kann, oder eine 2-Propenylengruppe,

$R^2$ ein Wasserstoffatom oder eine Alkylgruppe mit 1 bis 4 Kohlenstoffatomen,

$R^3$ bis $R^6$ jeweils ein Wasserstoffatom,

$R^7$ eine gegebenenfalls in 4-Stellung durch ein Fluor-, Chlor- oder Bromatom, durch eine Methyl-, Trifluormethyl-, Methoxy-, Phenyl- oder Nitrogruppe substituierte Phenylgruppe, eine 3,4-Dichlorphenyl-, 2,4-Dichlorphenyl-, 4-Chlor-3-methylphenyl-, 4-Amino-3-chlorphenyl-, 3,4-Dimethoxyphenyl-, 3,4-Methylendioxyphenyl-, 4-Amino-3,5-dichlorphenyl- oder eine 2-Naphthylgruppe, und

A eine Bindung, eine geradkettige oder verzweigte Alkylengruppe mit 1 bis 5 Kohlenstoffatomen oder eine Alkenylengruppe mit 2 oder 3 Kohlenstoffatomen bedeuten,

wobei alle vorstehend erwähnten Alkyl- und Alkoxyteile, sofern nichts anderes erwähnt wurde, 1 bis 3 Kohlenstoffatome enthalten können, deren Enantiomere, Diastereomere und geometrischen Isomere sowie deren Salze, insbesondere für die pharmazeutische Anwendung deren physiologisch verträgliche Salze mit anorganischen oder organischen Säuren,
insbesondere jedoch die Verbindungen

(1) trans-O-(p-Tolylacetyl)-4-(4-dimethylaminomethylphenyl)-cyclohexanol und

(2) trans-O-Phenylacetyl-4-(4-dimethylaminomethylphenyl)-cyclohexanol,

und deren Salze.

Herstellungsverfahren:

Die Verbindungen der Formel I lassen sich bespielsweise nach folgenden Verfahren herstellen:

a) Durch Umsetzung eines 4-Phenylcycloalkanols der allgemeinen Formel II

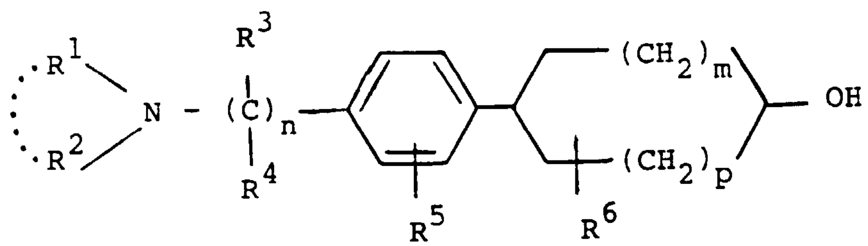

, (II)

in der
n, m, p und $R^1$ bis $R^6$ wie eingangs definiert sind, mit einer Carbonsäure oder deren reaktiven Derivaten der allgemeinen Formel III

$$R^7 - A - COX , \qquad (III)$$

in der
$R^7$ und A wie eingangs erwähnt definiert sind und X eine Hydroxygruppe oder eine reaktive Austrittsgruppe, z. B. ein Halogenatom, wie ein Chlor- oder Bromatom, eine Trimethylsilyloxygruppe, eine Sulfonyloxygruppe, z. B. die p-Toluolsulfonyloxygruppe, eine N-Heteroarylgruppe, z. B. die 1-Imidazolyl- oder die 1-Benzotriazolylgruppe, oder eine O-Isoharnstoffgruppe, z. B. die O-(N,N'-Dicyclohexyl)-isoharnstoffgruppe darstellt.

Die Umsetzung wird zweckmäßigerweise in einem Lösungsmittel wie Benzol, Toluol, Xylol, Di-Isopropylether, Dioxan, Tetrahydrofuran, Dimethylformamid, Dichlormethan oder Chloroform und gegebenenfalls in Gegenwart einer Base, wie beispielsweise Triethylamin, Pyridin oder 4-Dimethylaminopyridin, oder in Gegenwart einer Säure, insbesondere wenn X in der allgemeinen Formel III eine Hydroxygruppe darstellt, beispielsweise in Gegenwart von Bortrifluorid-Etherat oder eines sauren Kationenaustauschers bei einer Temperatur zwischen -10 und 150°C, vorzugsweise jedoch bei einer Temperatur zwischen -10 und 80°C, durchgeführt.

Falls die Reste $R^1$ und/oder $R^2$ freie Hydroxy-, Amino- oder Carboxygruppen besitzen, empfiehlt es sich, diese vor der Umsetzung in geeigneter Weise zu schützen, z. B. durch Überführung der Hydroxy- in eine Ethergruppe, z. B. die 2-Methoxyethoxymethyl-, die tert.-Butyl- oder die Benzylethergruppe, der Amino- in eine Carbamatgruppe, z. B. die Trichlorethyl-, 9-Fluorenylmethyl- oder die 2,4-Dichlorbenzylcarbamatgruppe und der Carboxyl- in eine Estergruppe, z. B. die 2,2,2-Trichlorethyl-, die tert.-Butyl- oder Benzylestergruppe, und die Schutzgruppen nach beendeter Umsetzung nach bekannten Methoden wieder abzuspalten.

b) Durch Umsetzung eines O-Acyl-4-phenylcycloalkanols der allgemeinen Formel IV

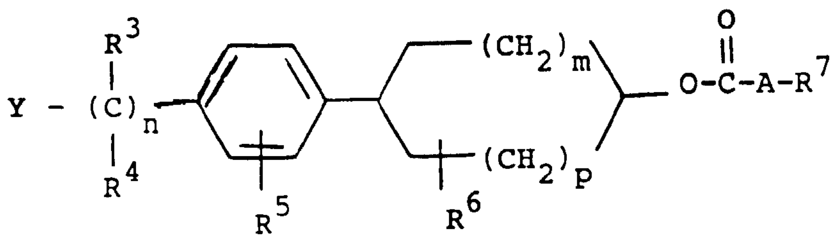

, (IV)

in der
n, m, p, $R^3$ bis $R^7$ und A wie eingangs definiert sind und Y eine reaktive Austrittsgruppe wie ein Halogenatom, z. B. ein Chlor- oder Bromatom, oder eine Sulfonyloxygruppe, z. B. die Methylsulfonyloxygruppe, darstellt, mit einem Amin der allgemeinen Formel V

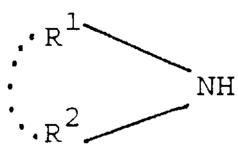

, (V)

in der
$R^1$ und $R^2$ wie eingangs definiert sind.

Die Umsetzung wird zweckmäßigerweise in einem geeigneten Lösungsmittel wie Ethanol-, tert.-Butanol, Dimethylformamid oder Tetrahydrofuran gegebenenfalls in Gegenwart einer Base, wie z. B. Kaliumcarbonat, Natriumethanolat, Kalium-tert.-butanolat oder Natriumhydrid, und gegebenenfalls unter Phasentransferbedingungen, bei einer Temperatur zwischen 0 und 100°C, durchgeführt.

c) Zur Herstellung von Verbindungen der allgemeinen Formel I, in der $R^1$ die eingangs erwähnten Bedeutungen besitzt und $R^2$ eine geradkettige oder verzweigte Alkylgruppe mit 1 bis 6 Kohlenstoffatomen, die durch eine Hydroxy-, Alkoxy-, Alkylcarbonyloxy-, Alkylcarbonylamino-, Carboxyl-, Alkoxycarbonyl-, Aminocarbonyl- oder Cyanogruppe substituiert sein kann, wobei die Hydroxy-, Alkoxy-, Alkylcarbonyloxy- oder Alkylcarbonylaminogruppe nicht am Kohlenstoffatom in Position 1 gebunden ist, darstellt:
Umsetzung eines O-Acyl-4-phenylcycloalkanols der allgemeinen Formel VI

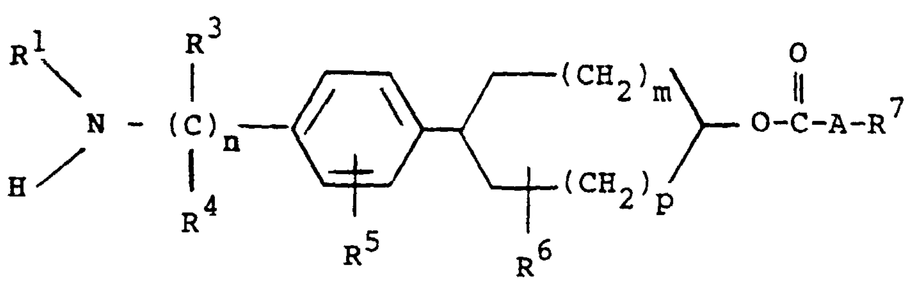

, (VI)

in der
n, m, p, $R^3$ bis $R^7$ und A wie eingangs erwähnt definiert sind und $R^1$ die oben erwähnten Bedeutungen besitzt, mit

einer Verbindung der allgemeinen Formel VII

$$R^{2'} - Z^1 , \qquad (VII)$$

in der
$R^{2'}$ eine geradkettige oder verzweigte Alkylgruppe mit 1 bis 6 Kohlenstoffatomen, die durch eine Hydroxy-, Alkoxy-, Alkylcarbonyloxy-, Alkylcarbonylamino-, Carboxyl-, Alkoxycarbonyl-, Aminocarbonyl- oder Cyanogruppe substituiert sein kann, wobei die Hydroxy-, Alkoxy-, Alkylcarbonyloxy- oder Alkylcarbonylaminogruppe nicht am Kohlenstoffatom in Position 1 gebunden ist, bedeutet und $Z^1$ eine reaktive Austrittsgruppe, wie beispielsweise ein Halogenatom, z. B. ein Chlor- oder ein Bromatom, oder eine Sulfonyloxygruppe, z. B. die Methylsulfonyloxygruppe, darstellt.

Die Umsetzung wird zweckmäßigerweise in einem Lösungsmittel oder Lösungsmittelgemisch wie beispielsweise Ethanol, tert.-Butanol, Tetrahydrofuran, Dimethylsulfoxid oder Dimethylformamid gegebenenfalls in Gegenwart eines säurebindenden Mittels, wie Natriumcarbonat, Kaliumcarbonat, Natriumhydroxid, Natriumhydrid, Natriummethanolat, Kalium-tert.-butanolat, Triethylamin oder Pyridin, wobei die beiden letzteren gleichzeitig auch als Lösungsmittel verwendet werden können, gegebenenfalls unter Phasentransferbedingungen, vorzugsweise bei Temperaturen zwischen 0 und 100°C, z.B. bei Temperaturen zwischen 20 und 50°C, durchgeführt.

Bei den vorstehend beschriebenen Umsetzungen können gegebenenfalls vorhandene reaktive Gruppen wie Hydroxy-, Amino-, Alkylamino-, Imino- oder Carboxylgruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.-Butyl-, 2-Methoxyethoxymethyl-, Benzyl- oder Tetrahydropyranylgruppe, als Schutzrest für eine Amino-, Alkylamino- oder Iminogruppe die Acetyl-, Benzoyl-, Ethoxycarbonyl- oder Benzylgruppe und als Schutzrest für eine Carboxylgruppe die 2,2,2-Trichlorethyl-, tert.-Butyl- oder Benzylestergruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Die nach den vorstehenden Verfahren hergestellten Verbindungen der allgemeinen Formel I lassen sich nach bekannten Methoden, z. B. Kristallisation oder Chromatographie reinigen und isolieren.

Desweiteren können die erhaltenen Verbindungen der allgemeinen Formel I gewünschtenfalls in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

In den erfindungsgemäßen Verbindungen der Formel I können entsprechend der Stellung der Substituenten am Cycloalkanring bzw. der Form der Substituenten $R^1$ bis $R^7$ Stereoisomere, wie Diastereomere, geometrische Isomere oder optische Isomere auftreten. Die Erfindung umfaßt sowohl die reinen Stereoisomeren als auch deren Mischungen.

Ausgangsverbindungen:

Die Ausgangsverbindungen der allgemeinen Formel II lassen sich beispielsweise auf folgendem Weg darstellen:

1. Durch Reduktion von 4-Phenylcycloalkanonen der allgemeinen Formel VIII

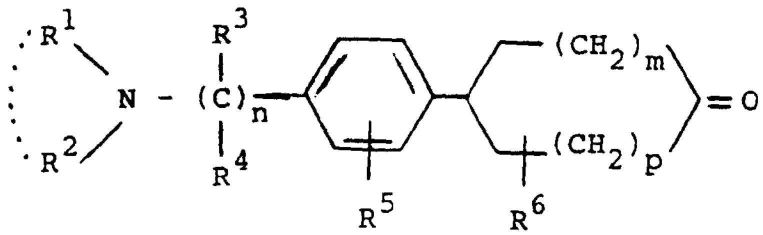

, (VIII)

in der
n, m, p und $R^1$ bis $R^6$ wie eingangs definiert sind.

Durch Auswahl geeigneter Reduktionsmittel, z. B. Natriumborhydrid bzw. Lithium-tri-sec.-butylborhydrid (L-Selectride) kann die Reaktion so gelenkt werden, daß vorwiegend das e,e-Isomere bzw. das e,a-Isomere einer Verbindung der allgemeinen Formel II entsteht.

Die Ketone der allgemeinen Formel VIII lassen sich nach bekannten Methoden herstellen, z. B. durch Umsetzung von Monoethylenketalen der allgemeinen Formel IX

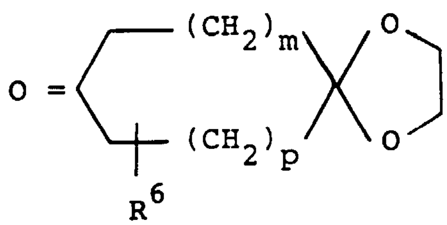

, (IX)

mit einer metallorganischen Verbindung der allgemeinen Formel X

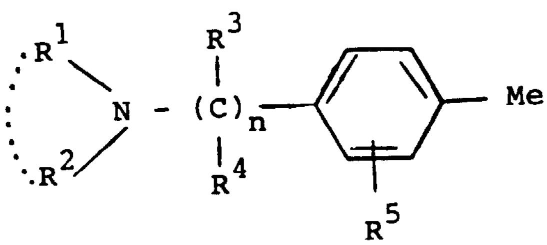

, (X)

in der
n und $R^1$ bis $R^5$ wie eingangs erwähnt definiert sind und Me ein Lithiumatom oder eine Gruppe -MgHal bedeutet, wobei Hal ein Halogenatom, vorzugsweise ein Chloratom darstellt, und anschließender Wasserabspaltung, Hydrierung der entstandenen Doppelbindung und Hydrolyse der Ketalgruppierung.

Dabei kann das Verfahren so abgewandelt werden, daß ein Keton der allgemeinen Formel VIII, in der $R^6$ ein Wasserstoffatom darstellt, nach Durchführung der vorstehend beschriebenen Reaktionsfolge in ein Keton der allgemeinen Formel VIII, in der $R^6$ eine Alkylgruppe mit 1 bis 4 Kohlenstoffatomen darstellt, übergeführt wird, z. B. durch Alkylierung des Keton-Enolat-Ions.

Eine weitere Darstellungsmethode für Verbindungen der allgemeinen Formel VIII besteht in der Dieckmann-Cyclisierung von Dicarbonsäureestern der allgemeinen Formel XI

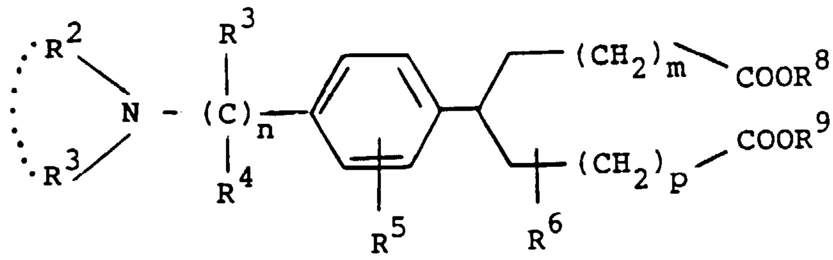

, (XI)

in der

n, m, p und $R^1$ bis $R^6$ wie eingangs definiert sind und $R^8$ und $R^9$, die gleich oder verschieden sein können, eine Alkyl-, Aralkyl- oder Arylgruppe bedeutet, und anschließender Verseifung und Decarboxylierung nach bekannten Methoden.

2. Die Ausgangsverbindungen der allgemeinen Formel IV lassen sich beispielsweise durch Halomethylierung von O-Acyl-4-phenylcycloalkanolen der allgemeinen Formel XII,

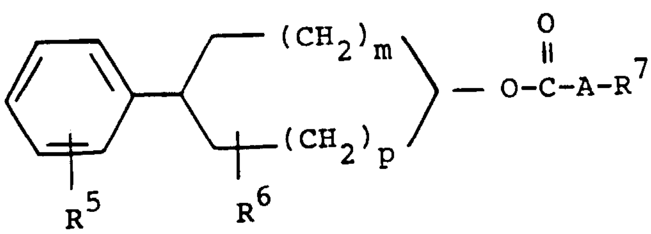

, (XII)

in der

m, p, $R^5$ bis $R^7$ und A wie eingangs erwähnt definiert sind, mit einem entsprechenden Aldehyd und Halogenwasserstoff, z. B. Chlor- oder Bromwasserstoff, in Gegenwart eines Friedel-Crafts-Katalysators, z. B. Zinkchlorid, und gewünschtenfalls anschließender Substitution des Halogenatoms durch eine geeignete andere reaktive Austrittsgruppe herstellen.

3. Die Ausgangsverbindungen der allgemeinen Formel VI lassen sich beispielsweise aus O-Acyl-4-phenyl-cycloalkanolen der allgemeinen Formel XIII

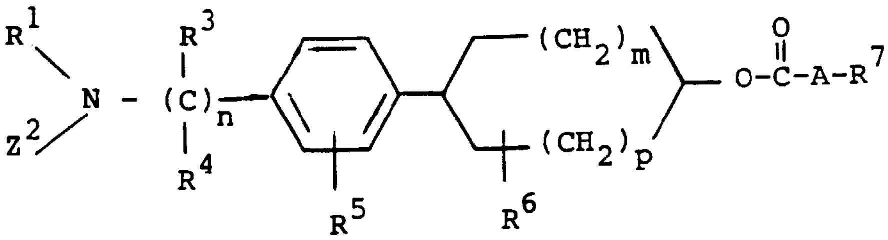

, (XIII)

in der

n, m, p, $R^1$, $R^3$ bis $R^7$ und A wie eingangs definiert sind und $Z^2$ eine geeignete Schutzgruppe bedeutet, durch Abspaltung dieser Schutzgruppe herstellen. Als Schutzgruppe kommt beispielsweise die tert.-Butoxycarbonyl-, die 1-(3,5-Di-tert.-butylphenyl)-1-methylethoxycarbonyl- oder die 2-(4-Pyridyl)ethoxycarbonylgruppe in Betracht. Die Verbindungen der allgemeinen Formel XIII sind beispielsweise analog dem in Verfahren 1 beschriebenen Weg synthetisierbar.

Die Ausgangsverbindungen der Formeln III und V sind literaturbekannt oder lassen sich nach literaturbekannten Methoden erhalten.

Die Verbindungen der allgemeinen Formel I besitzen interessante biologische Eigenschaften. Sie stellen Inhibitoren der Cholesterolbiosynthese, insbesondere Inhibitoren des Enzyms 2,3-Epoxisqualen-Lanosterol-Cyclase dar. Aufgrund ihrer biologischen Eigenschaften sind sie besonders geeignet zur Behandlung und Prophylaxe von Hyperlipidämien, insbesondere der Hypercholesterolämie, der Hyperlipoproteinämie und der Hypertriglyceridämie und den daraus resultierenden atherosklerotischen Gefäßveränderungen mit ihren Folgeerkrankungen wie koronare Herzkrankheit, cerebrale Ischämie, Claudicatio intermittens, Gangrän und andere.

Zur Behandlung dieser Erkrankungen können die Verbindungen der allgemeinen Formel I dabei entweder alleine zur Monotherapie eingesetzt werden oder in Kombination mit anderen cholesterol- oder lipidsenkenden Substanzen zur Anwendung gelangen, wobei die Verbindungen vorzugsweise als orale Formulierung, gegebenenfalls auch als rektale Formulierung verabreicht werden können. Als Kombinationspartner kommen dabei beispielsweise in Frage:

- gallensäurebindende Harze wie z. B. Cholestyramin, Cholestipol und andere,
- Verbindungen, die die Cholesterolresorption hemmen, wie z. B. Sitosterol und Neomycin,
- Verbindungen, die in die Cholesterolbiosynthese eingreifen, wie z. B. HMG-CoA-Reduktaseinhibitoren wie Lovastatin, Simvastatin, Pravastatin und andere,
- Squalen-Epoxidaseinhibitoren wie beispielsweise NB 598 und analoge Verbindungen sowie
- Squalen-Synthetaseinhibitoren wie beispielsweise Vertreter der Klasse der Isoprenoid-(phosphinylmethyl)phosphonate und Squalestatin.

Als weitere mögliche Kombinationspartner sind noch zu erwähnen die Klasse der Fibrate, wie Clofibrat, Bezafibrat, Gemfibrozil und andere, Nikotinsäure, ihre Derivate und Analoge wie beispielsweise Acipimox sowie Probucol.

Desweiteren sind die Verbindungen der allgemeinen Formel I geeignet zur Behandlung von Erkrankungen, die mit überhöhter Zellproliferation im Zusammenhang stehen. Cholesterol ist ein essentieller Zellbestandteil und muß für die Zellproliferation, d. h. Zellteilung, in ausreichender Menge vorhanden sein. Die Inhibierung der Zellproliferation durch Inhibierung der Cholesterolbiosynthese ist am Beispiel der glatten Muskelzellen mit dem HMG-CoA-Reduktaseinhibitor des Statintyps Lovastatin, wie eingangs erwähnt, beschrieben.

Als Beispiele für Erkrankungen, die mit überhöhter Zellproliferation zusammenhängen sind zunächst Tumorerkrankungen zu nennen. In Zellkultur- und in-vivo-Experimenten wurde gezeigt, daß die Senkung des Serumcholesterols oder der Eingriff in die Cholesterolbiosynthese durch HMG-CoA-Reduktaseinhibitoren das Tumorwachstum vermindert (Lancet 339, 1154-1156 [1992]). Die erfindungsgemäßen Verbindungen der Formel I sind deshalb aufgrund ihrer cholesterolbiosyntheseinhibitorischen Wirkung potentiell für die Behandlung von Tumorerkrankungen geeignet. Sie können dabei alleine oder zur Unterstützung bekannter Therapieprinzipien Verwendung finden.

Als weitere Beispiele sind hyperproliferative Hauterkrankungen wie beispielsweise Psoriasis, Basalzellkarzinome, Plattenepithelkarzinome, Keratosis und Keratinisierungsstörungen zu nennen. Der hier verwendete Ausdruck "Psoriasis" bezeichnet eine hyperproliferative Hauterkrankung, die den Regulierungsmechanismus der Haut verändert. Insbesondere werden Läsionen gebildet, die primäre und sekundäre Veränderungen der Proliferation in der Epidermis, entzündliche Reaktionen der Haut und die Expression regulatorischer Moleküle wie Lymphokine und Entzündungsfaktoren beinhalten. Psoriatische Haut ist morphologisch durch einen verstärkten Umsatz von Epidermiszellen, verdickte Epidermis, abnormale Keratinisierung entzündlicher Zellinfiltrate in die Dermisschicht und polymorphonucleäre Leukozyteninfiltration in die Epidermis, die eine Zunahme des Basalzellzyklus bedingt, gekennzeichnet. Zusätzlich sind hyperkeratotische und parakeratotische Zellen anwesend.

Der Ausdruck "Keratosis", "Basalzellkarzinome", "Plattenepithelkarzinome" und "Keratinisierungsstörungen" bezieht sich auf hyperproliferative Hauterkrankungen, bei denen der Regulierungsmechanismus für die Proliferation und Differenzierung der Hautzellen unterbrochen ist.

Die Verbindungen der Formel I sind wirksam als Antagonisten der Hauthyperproliferation, d. h. als Mittel, die die Hyperproliferation menschlicher Keratinozyten hemmen. Infolgedessen sind sie als Mittel zur Behandlung hyperproliferativer Hauterkrankungen wie Psoriasis, Basalzellkarzinomen, Keratinisierungsstörungen und Keratosis geeignet. Zur Behandlung dieser Krankheiten können die Verbindungen der Formel I entweder oral oder topisch appliziert werden, wobei sie entweder alleine in Form der Monotherapie oder in Kombination mit bekannten Wirkstoffen eingesetzt werden können.

Des weiteren zu nennen sind durch chirurgische Maßnahmen wie PTCA (perkutane transluminale coronare Angioplastie) oder Bypass-Operationen ausgelöste hyperproliferative Gefäßerkrankungen wie Stenosen und Gefäßverschlüsse, die auf der Proliferation glatter Muskelzellen beruhen. Wie eingangs erwähnt läßt sich diese Zellproliferation bekanntlich durch HMG-CoA-Reduktaseinhibitoren vom Statintyp, wie Lovastatin, unterdrücken. Aufgrund ihrer inhibitorischen Wirkung auf die Cholesterolbiosynthese sind auch die Verbindungen der allgemeinen Formel I geeignet

zur Behandlung und Prophylaxe dieser Erkrankungen, wobei sie entweder alleine oder in Kombination mit bekannten Wirkstoffen, wie z. B. intravenös appliziertes Heparin, vorzugsweise in oraler Applikation Verwendung finden können.

Eine weitere Einsatzmöglichkeit der erfindungsgemäßen Verbindungen der allgemeinen Formel I ist die Prophylaxe und Behandlung von Gallensteinleiden. Die Gallensteinbildung wird ausgelöst durch ein ungünstiges Cholesterol-Gallensäure-Verhältnis in der Gallenflüssigkeit, wodurch die Löslichkeit des Cholesterols überschritten wird und es zur Ausfällung des Cholesterols in Form von Gallensteinen kommt. Die Wirksamkeit des HMG-CoA-Reduktase-Inhibitors Lovastatin bei der Auflösung von Gallensteinen, insbesondere in Kombination mit Ursodeoxycholsäure, ist beschrieben in Gastroenterology 102, No. 4, Pt.2, A319 [1992]. Aufgrund ihrer Wirkungsweise sind die Verbindungen der allgemeinen Formel I deshalb auch für die Prophylaxe und Behandlung von Gallensteinleiden von Bedeutung. Sie können dabei entweder allein oder in Kombination mit bekannten Therapien wie beispielsweise der Behandlung mit Ursodeoxycholsäure oder der Schockwellenlithotripsie vorzugsweise in oraler Applikation Verwendung finden.

Schließlich sind die Verbindungen der allgemeinen Formel I geeignet zur Therapie von Infektionen durch pathogene Pilze wie z. B. Candida albicans, Aspergillus niger, Trichophyton mentagrophytes, Penicillium sp., Cladosporium sp. und andere. Wie bereits eingangs erwähnt ist das Endprodukt der Sterolbiosynthese im Pilzorganismus nicht Cholesterol, sondern das für die Integrität und Funktion der Pilzzellmembranen essentielle Ergosterol. Die Inhibierung der Ergosterolbiosynthese führt deshalb zu Wachstums störungen und gegebenenfalls zur Abtötung der Pilzorganismen.

Zur Behandlung von Mykosen können die Verbindungen der allgemeinen Formel I entweder oral oder topisch appliziert werden. Dabei können sie entweder alleine oder in Kombination mit bekannten antimykotischen Wirkstoffen eingesetzt werden, insbesondere mit solchen, die in andere Stufen der Sterolbiosynthese eingreifen, wie beispielsweise den Squalen-Epoxidasehemmern Terbinafin und Naftifin oder den Lanosterol-14$\alpha$-Demethylaseinhibitoren vom Azol-Typ wie beispielsweise Ketoconazol und Fluconazol.

Eine weitere Verwendungsmöglichkeit der Verbindungen der allgemeinen Formel I betrifft die Anwendung in der Geflügelhaltung. Die Senkung des Cholesterolgehaltes von Eiern durch Verabreichung des HMG-CoA-Reduktaseinhibitors Lovastatin an Legehennen ist beschrieben (FASEB Journal 4, A 533, Abstracts 1543 [1990]). Die Erzeugung cholesterolarmer Eier ist von Interesse, da die Cholesterolbelastung des Körpers durch Eier mit reduziertem Cholesterolgehalt ohne eine Änderung der Ernährungsgewohnheiten vermindert werden kann. Aufgrund ihrer inhibitorischen Wirkung auf die Cholesterolbiosynthese können die Verbindungen der allgemeinen Formel I auch in der Geflügelzucht zur Erzeugung cholesterolarmer Eier Verwendung finden, wobei die Substanzen vorzugsweise als Zusatz zum Futter verabreicht werden.

Die biologische Wirkung von Verbindungen der allgemeinen Formel I wurden nach folgenden Methoden bestimmt:

I. Messung der Hemmung des $^{14}C$-Acetat-Einbaus in die mit Digitonin fällbaren Steroide:

Methode:

Humane Hepatoma-Zellen (HEP-G2) werden nach 3-tägiger Anzucht für 16 Stunden in cholesterolfreiem Medium stimuliert. Die zu testenden Substanzen (gelöst in Dimethylsulfoxid, Endkonzentration 0,1%) werden während dieser Stimulationsphase zugesetzt. Anschließend wird nach Zugabe von 200 $\mu$Mol/l 2-$^{14}C$-Acetat für weitere zwei Stunden bei 37°C im Brutschrank weiterinkubiert.

Nach Ablösung der Zellen und Verseifen der Sterolester wird nach Extraktion Digitonin zugesetzt und die ausgefällten Sterole isoliert. Das in die digitoninfällbaren Sterole eingebaute $^{14}C$-Acetat wird durch Szintillationsmessung bestimmt.

Die Untersuchung der Hemmwirkung wurde bei Testkonzentrationen von $10^{-7}$ Mol/l und $10^{-8}$ Mol/l durchgeführt. Es wurde gefunden, daß beispielsweise die folgenden Verbindungen A bis Q der allgemeinen Formel I bei diesen Testkonzentrationen eine gute Hemmwirkung zeigen, z. B. bei einer Testkonzentration von $10^{-8}$ Mol/l eine Hemmwirkung von mindestens 50%:

A = cis-O-(4-Chlorbenzoyl)-4-(4-dimethylaminomethylphenyl)cyclohexanol

B = cis-O-(4-Phenyl-3-butenoyl)-4-(4-dimethylaminomethylphenyl)-cyclohexanol

C = trans-O-(4-Chlorphenylacetyl)-4-(4-dimethylaminomethylphenyl)-cyclohexanol

D = cis-O-(5-Methylhexanoyl)-4-(4-dimethylaminomethylphenyl)-cyclohexanol

E = trans-O-(2-Phenylpropionyl)-4-(4-dimethylaminomethylphenyl)-cyclohexanol

F = trans-O-(4-Fluorphenylacetyl)-4-(4-dimethylaminomethylphenyl)-cyclohexanol

G = trans-O-(3,4-Dichlorphenylacetyl)-4-(4-dimethylaminomethylphenyl)-cyclohexanol

H = cis-O-(4-Fluorcinnamoyl)-4-(4-dimethylaminomethylphenyl)cyclohexanol

I = trans-O-(p-Tolylacetyl)-4-(4-dimethylaminomethylphenyl)cyclohexanol

J = trans-O-(4-[Trifluormethyl]-phenylacetyl)-4-(4-dimethylaminomethylphenyl)-cyclohexanol

K = trans-O-(2-Naphthylacetyl)-4-(4-dimethylaminomethylphenyl)-cyclohexanol

L = trans-O-(4-Nitrophenylacetyl)-4-(4-dimethylaminomethylphenyl)-cyclohexanol

M = trans-O-(4-Bromphenylacetyl)-4-(4-dimethylaminomethylphenyl)-cyclohexanol

N = trans-O-(2,4-Dichlorphenylacetyl)-4-(4-dimethylaminomethylphenyl)-cyclohexanol

O = trans-O-([4-Amino-3-chlorphenyl]acetyl)-4-(4-dimethylaminomethylphenyl)-cyclohexanol

P = trans-O-(4-Methoxyphenylacetyl)-4-(4-dimethylaminomethylphenyl)-cyclohexanol

Q = trans-O-(4-Chlorphenylacetyl)-4-(4-methylaminomethylphenyl)-cyclohexanol.

Die Prozentwerte, um die die obigen Verbindungen den $^{14}C$-Acetat-Einbau hemmen, sind in der folgenden Tabelle angegeben:

| Mol/l | $10^{-7}$ | $10^{-8}$ |
|---|---|---|
| A | -85 | -51 |
| B | -87 | -58 |
| C | -83 | -66 |
| D | -88 | -53 |
| E | -89 | -72 |
| F | -86 | -66 |
| G | -89 | -74 |
| H | -86 | -51 |
| I | -90 | -72 |
| J | -89 | -87 |
| K | -86 | -54 |
| L | -83 | -67 |
| M | -84 | -64 |
| N | -85 | -67 |
| O | -79 | -51 |
| P | -73 | -52 |
| Q | -79 | -50 |

Wie eingangs erwähnt, sind in der Literatur vereinzelt Inhibitoren des Enzyms 2,3-Epoxisqualen-Lanosterol-Cyclase beschrieben, die sich jedoch strukturell sehr stark von den erfindungsgemäßen Verbindungen der Formel I unterscheiden. Die zu den Verbindungen der allgemeinen Formel I strukturell nächstverwandten Verbindungen sind in der EP 0 468 457 beschrieben. Zum Vergleich wurde deshalb das Beispiel 1 dieser Publikation nach der oben beschriebenen Bestimmungsmethode in Testkonzentrationen von $10^{-5}$ Mol/l und $10^{-6}$ Mol/l geprüft. Die dabei gefundenen Hemmwerte von 41% bzw. 13% zeigen, daß diese Verbindungen den erfindungsgemäßen Verbindungen der allgemeinen Formel I deutlich unterlegen sind.

II. Messung der in-vivo-Wirkung an der Ratte nach oraler Gabe

Die Inhibierung des Enzyms 2,3-Epoxisqualen-Lanosterol-Cyclase bewirkt eine Erhöhung der 2,3-Epoxisqualenspiegel in Leber und Plasma. Die Menge an gebildetem 2,3-Epoxisqualen dient daher als direktes Maß für die Wirkstärke am Ganztier. Die Bestimmung wird nach folgender Methode durchgeführt:

Männlichen Wistar-Ratten (160-190 g Körpergewicht) wird die in 1,5%iger wässriger Methylcellulose suspendierte Prüfsubstanz via Schlundsonde appliziert. 5 Stunden nach Applikation wird Blut retroorbital aus dem Venenplexus gewonnen. Plasma wird nach der Methode von Bligh und Dyer (Canad. J. Biochem. Physiol. 37, 912, [1959]) aufgearbeitet, über eine Vorsäule gereinigt und danach mittels HPLC analysiert. Die erhaltenen Peaks werden über Eichsubstanzen identifiziert und quantifiziert. Ein interner Standard dient der Überprüfung der Reproduzierbarkeit der Ergebnisse.

Die Untersuchungen wurden mit Konzentrationen von 0,1 und 1,0 mg/kg durchgeführt. In der folgenden Tabelle sind beispielhaft die Testdaten der vorstehend erwähnten Substanzen B, C, J, M, N und P für die erzielten 2,3-Epoxisqualen-Spiegel in Rattenplasma aufgeführt. Bei den Kontrolltieren treten unter den Versuchsbedingungen keine meßbaren 2,3-Epoxisqualen-Spiegel auf.

2,3-Epoxisqualen-Spiegel in Plasma (Ratte)

| 2,3-Epoxisqualen [µg/ml] | | |
|---|---|---|
| Substanz | 0,1 mg/kg | 1,0 mg/kg |
| B | 0,4 | 1,1 |
| C | 0,6 | 4,2 |
| J | 0,5 | 3,6 |
| M | 0,6 | 3,5 |
| N | 0,1 | 2,2 |
| P | 0,3 | 0,9 |

Von keinem der in der Literatur beschriebenen Inhibitoren des Enzyms 2,3-Epoxisqualen-Lanosterol-Cyclase ist bisher eine Inhibierung der Cholesterolbiosynthese am Ganztier beschrieben.

Die Verbindungen erwiesen sich in der kurativen Dosis als völlig untoxisch. Beispielsweise zeigte die Verbindung C an der Ratte, die Verbindungen J und M an der Maus nach oraler Applikation von 100 mg/kg, einmal täglich über 5 Tage, keine Nebeneffekte.

Zur pharmazeutischen Anwendung lassen sich die Verbindungen der allgemeinen Formel I in an sich bekannter Weise in die üblichen pharmazeutischen Zubereitungsformen für die orale und topische Verabreichung einarbeiten.

Formulierungen für die orale Verabreichung umfassen beispielsweise Tabletten, Dragees und Kapseln, für die rektale Verabreichung kommen vorzugsweise Suppositorien in Betracht.

Topische Formulierungen umfassen Gele, Cremes, Lotionen, Salben, Puder, Aerosole und andere herkömmliche Formulierungen zur Anwendung von Heilmitteln auf der Haut. Die Wirkstoffmenge für die topische Anwendung beträgt 1 bis 50 mg pro Gramm Formulierung, vorzugsweise jedoch 5 bis 20 mg pro Gramm Formulierung. Neben der Anwendung auf der Haut können die topischen Formulierungen der vorliegenden Erfindung auch angewandt werden bei der Behandlung von Schleimhäuten, die der topischen Behandlung zugänglich sind. Beispielsweise können die topischen Formulierungen auf die Schleimhäute des Mundes, des unteren Colons und andere aufgebracht werden.

Die orale oder rektale Tagesdosis beträgt zwischen 1 und 1200 mg für einen Menschen mit 60 kg Körpergewicht, bevorzugt ist jedoch eine Tagesdosis von 5 bis 100 mg für einen Menschen mit 60 kg Körpergewicht. Die Tagesdosis wird vorzugsweise in 1 bis 3 Einzelgaben aufgeteilt.

Bei topischer Anwendung können die Verbindungen in Zubereitungen, die etwa 1 bis 1000 mg, insbesondere 10 bis 300 mg Wirkstoff pro Tag enthalten, verabreicht werden. Die Tagesdosis wird vorzugsweise in 1 bis 3 Einzelgaben aufgeteilt.

Zur Anwendung in der Geflügelzucht zur Erzeugung cholesterolarmer Eier werden die Wirkstoffe der allgemeinen Formel I den Tieren nach den üblichen Methoden als Zusatz zu geeigneten Futtermitteln verabreicht. Die Konzentration der Wirkstoffe im Fertigfutter beträgt normalerweise 0,01 bis 1%, vorzugsweise jedoch 0,05 bis 0,5%.

Die Wirkstoffe können als solche dem Futter zugesetzt werden. So enthalten die erfindungsgemäßen Futtermittel für Legehennen neben dem Wirkstoff und gegebenenfalls neben einer üblichen Vitamin-Mineral-Mischung beispielsweise Mais, Sojabohnenmehl, Fleischmehl, Futterfett und Sojaöl. Zu diesem Futter wird eine der eingangs erwähnten Verbindungen der Formel I als Wirkstoff in einer Konzentration von 0,01 bis 1%, vorzugsweise jedoch 0,05 bis 0,5% zugemischt.

Die nachstehenden Beispiele sollen die Erfindung näher erläutern:

In den nachfolgenden Beispielen wurden die dünnschichtchromatographischen Untersuchungen mit DC-Fertigplatten der Firma E. Merck, Darmstadt durchgeführt und zwar an:

a) Kieselgel 60 $F_{254}$
b) Aluminiumoxid $F_{254}$ (Typ E)

Herstellung der Ausgangsverbindungen:

Beispiel I

4-(4-Dimethylaminomethylphenyl)-cyclohexanon

a) 4-(4-Dimethylaminomethylphenyl)-4-hydroxycyclohexanonethylenketal

Zu einer auf -70°C gekühlten Lösung von 36,4 g (0,17 Mol) 4-Brom-N,N-dimethylbenzylamin in 250 ml trokkenem Tetrahydrofuran werden unter Stickstoffatmosphäre und unter Rühren 112 ml (0,179 Mol) einer 1,6-molaren Lösung von n-Butyllithium in Hexan so zugetropft, daß die Temperatur -65°C nicht übersteigt. Die orangefarbene Lösung wird weitere 15 Minuten bei -70°C gerührt und dann innerhalb von 10 Minuten mit einer Lösung von 27,6 g (0,172 Mol) 1,4-Cyclohexandion-monoethylenketal in 110 ml Tetrahydrofuran versetzt, wobei die Temperatur -65°C nicht übersteigen darf.

Die Reaktionsmischung wird zunächst 30 Minuten bei -70°C und dann ohne Außenkühlung gerührt, bis +20°C erreicht sind, in 600 ml Eiswasser gegossen und mit 200 ml Ethylacetat extrahiert. Die organische Phase wird abgetrennt und die wäßrige Phase mehrmals mit Ethylacetat extrahiert. Die vereinigten organischen Extrakte werden mit Natriumsulfat getrocknet, im Vakuum eingeengt und der verbleibende Rückstand wird aus Diisopropylether umkristallisiert. Es werden 41,9 g (85% der Theorie) 4-(4-Dimethylaminomethylphenyl)-4-hydroxycyclohexanon-ethylenketal vom Schmelzpunkt 84-86°C erhalten.

b) 1-(4-Dimethylaminomethyl)phenyl-4-ethylendioxy-1-cyclohexen

Eine Mischung aus 22,4 g (0,077 Mol) 4-(4-Dimethylaminomethylphenyl)-4-hydroxycyclohexanon-ethylenketal, 15,0 g (0,079 Mol) p-Toluolsulfonsäure-Monohydrat, 39 ml Ethylenglykol und 240 ml Toluol wird unter Rühren 3,5 Stunden zum Rückfluß erhitzt und das entstehende Reaktionswasser kontinuierlich entfernt. Die abgekühlte Reaktionsmischung wird in 200 ml Wasser gegossen und mit 2n NaOH auf pH 12-13 gestellt. Die organische Phase wird abgetrennt und die wäßrige Phase mehrmals mit Toluol extrahiert. Die vereinigten organischen Extrakte werden mit Natriumsulfat getrocknet und im Vakuum eingeengt. Man erhält 21 g (ca. 100%) der Titelverbindung als gelbes Öl.

c) 1-(4-Dimethylaminomethyl)phenyl-4-ethylendioxy-1-cyclohexan

Eine Lösung von 21 g (0,077 Mol) des rohen 1-(4-Dimethylaminomethyl)phenyl-4-ethylendioxy-l-cyclohexen in 200 ml Ethylacetat und 100 ml Methanol wird mit 5 g Palladium/Bariumsulfat-Katalysator versetzt und 1,5 Stunden bei einem Wasserstoffdruck von 5 bar hydriert. Nach Abtrennen des Katalysators wird im Vakuum eingeengt. Man erhält 20 g (ca. 100%) der Titelverbindung als gelb-braunes Öl.

d) 4-(4-Dimethylaminomethyl)phenyl-cyclohexanon

Ein Gemisch aus 20 g (0,077 Mol) des rohen 1-(4-Dimethylaminomethyl)phenyl-4-ethylendioxy-1-cyclohexan und 110 ml 2N-Salzsäure wird 3,5 Stunden bei Raumtemperatur gerührt.

Die entstandene wäßrige Lösung wird mehrmals mit Ethylacetat extrahiert; die organischen Extrakte werden verworfen. Die Wasserphase wird unter Kühlung mit 50%-iger Natronlauge auf pH 13-14 gestellt und erneut mehrmals mit Ethylacetat extrahiert. Die vereinigten organischen Extrakte werden mit gesättigter Natriumchloridlösung gewaschen, mit Natriumsulfat getrocknet und im Vakuum eingeengt. Es werden 14 g (79% der Theorie) 4-(4-Dimethylaminomethyl)phenyl-cyclohexanon vom Schmelzpunkt 64-67°C als hellgelbes Produkt erhalten. Eine analytische Probe wird aus Petrolether 60/90 umkristallisiert.

Schmelzpunkt: 65-67°C

| $C_{15}H_{21}NO$ (231,34) | | | |
|---|---|---|---|
| Ber.: | C 77,88 | H 9,15 | N 6,05 |
| Gef.: | 77,69 | 9,32 | 5,98 |

Beispiel II

trans-4-(4-Dimethylaminomethylphenyl)-cyclohexanol

Zu einer auf -10°C gekühlten Lösung von 11,1 g (0,048 Mol) 4-(4-Dimethylaminomethylphenyl)-cyclohexanon in 100 ml absolutem Methanol wird unter Rühren portionsweise 1,82 g (0,048 Mol) Natriumborhydrid gegeben. Man läßt die Reaktionsmischung 1,5 Stunden bei Raumtemperatur nachreagieren und engt anschließend im Vakuum ein. Der verbleibende Rückstand wird mit Wasser versetzt, mit konzentrierter Salzsäure angesäuert, 30 Minuten bei Raumtemperatur gerührt, mit 50%-iger Natronlauge alkalisch gestellt und mehrmals mit Chloroform extrahiert. Die vereinigten Extrakte werden mit Natriumsulfat getrocknet und im Vakuum eingeengt. Der verbleibende Rückstand, der aus einem Gemisch von trans/cis-4-(4-Dimethylaminomethylphenyl)-cyclohexanol besteht (cis-Anteil < 10%) wird säulenchromatographisch gereinigt (Aluminiumoxid neutral, Aktivitätsstufe III, ICN; Petrolether/ Methylethylketon = 5:1).

Es werden weiße Kristalle vom Schmelzpunkt 63-65°C erhalten. Ausbeute: 8,8 g (79% der Theorie).

| $C_{15}H_{23}NO$ (233,36) | | | |
|---|---|---|---|
| Ber.: | C 77,21 | H 9,93 | N 6,00 |
| Gef.: | 77,34 | 10,02 | 5,89 |

Beispiel III

cis-4-(4-Dimethylaminomethylphenyl)-cyclohexanol

50 ml (0,05 Mol) einer 1-molaren Lösung von Lithium-tri-sec.-butylborhydrid in absolutem Tetrahydrofuran werden unter Stickstoffatmosphäre mit 100 ml absolutem Tetrahydrofuran verdünnt und anschließend bei -65°C bis -70°C unter Rühren innerhalb von 10 Minuten mit einer Lösung von 5,8 g (0,025 Mol) 4-(4-Dimethylaminomethylphenyl)-cyclohexanon in 50 ml absolutem Tetrahydrofuran versetzt. Anschließend läßt man die Reaktionsmischung 3 Stunden bei -70°C reagieren und erwärmt dann innerhalb 1 Stunde auf Raumtemperatur. Danach wird mit 20 ml 75%-igem wäßrigem Ethanol hydrolysiert und das Organoboran mit alkalischem Wasserstoffperoxid (10 ml 6 M NaOH/15 ml 30% $H_2O_2$) oxidiert. Die organische Phase wird abgetrennt, die wäßrige Phase mit Kaliumcarbonat gesättigt und mit 50 ml Ethylacetat extrahiert. Die vereinigten organischen Extrakte werden mit Natriumsulfat getrocknet und im Vakuum eingeengt. Der verbleibende schmierige Rückstand, der aus einem Gemisch von cis/trans-4-(4-Dimethylaminomethylphenyl)-cyclohexanol besteht (trans-Anteil < 5%) wird saulenchromatographisch gereinigt (Aluminiumoxid neutral, Aktivitätsstufe III, ICN; Petrolether/Methylethylketon = 5:1). Das Produkt fällt als farbloses Öl an.
Ausbeute: 4,1 g (71% der Theorie).
$^1$H-NMR-Spektrum (200 MHz, $CDCl_3$); Signale bei ppm:
1,5-2,0 (2m,8H); 2,25 (s,6H); 2,4-2,65 (m,1H); 3,4 (s,2H); 4,1-4,18 (m,1H); 7,15-7,3 (m,4H).

Beispiel IV

trans-O-Acetyl-4-(4-chlormethylphenyl)-cyclohexanol

a) 4-Phenylcyclohexanol

Zu einer auf -10°C gekühlten Lösung von 31,4 g (0,18 Mol) 4-Phenylcyclohexanon in 500 ml absolutem Methanol werden unter Rühren portionsweise 6,8 g (0,18 Mol) Natriumborhydrid gegeben. Man läßt die Reaktionsmischung 0,5 Stunden bei -10°C und 3 Stunden bei Raumtemperatur reagieren und engt dann im Vakuum ein. Der verbleibende Rückstand wird mit Wasser versetzt und mit 2N Salzsäure angesäuert. Die entstandene Suspension wird 1 Stunde gerührt und das kristalline Produkt abgesaugt, getrocknet und aus Diisopropylether umkristallisiert. Es werden 21 g (66% der Theorie) 4-Phenylcyclohexanol vom Schmelzpunkt 112-114°C erhalten.

b) O-Acetyl-4-phenylcyclohexanol

Zu einer Mischung aus 20,3 g (0,115 Mol) 4-Phenylcyclohexanol, 14,2 ml (0,15 Mol) Acetanhydrid und 29 ml Triethylamin werden bei Raumtemperatur unter Rühren 2,3 g (0,02 Mol) 4-Dimethylaminopyridin gegeben, wobei unter Exothermie eine klare Lösung entsteht. Es wird 3 Stunden auf 80°C erwärmt und das Reaktionsgemisch anschließend in Eiswasser gegossen. Das ausgefallene kristalline Produkt wird abgesaugt, in Ether gelöst, mit Natriumbikarbonat-Lösung gewaschen, getrocknet und im Vakuum eingeengt. Es werden 23 g (92% der Thorie) O-Acetyl-4-phenylcyclohexanol erhalten. Das Produkt fällt zunächst als Öl an, kristallisiert jedoch beim Stehenlassen.

Schmelzpunkt: 43-45°C.

c) trans-O-Acetyl-4-(4-chlormethylphenyl)-cyclohexanol

Eine Lösung von 24,3 g (0,11 Mol) O-Acetyl-4-phenylcyclohexanol in 1300 ml Methylenchlorid wird mit 26,0 g (0,86 Mol) Paraformaldehyd und 26,0 g (0,19 Mol) Zinkchlorid versetzt. In diese Suspension wird unter Rühren während 2,5 Stunden Chlorwasserstoff eingeleitet, wobei die Temperatur auf ca. 30°C ansteigt und eine weitgehend homogene Lösung ensteht. Anschließend läßt man das Gemisch 15 Stunden bei Raumtemperatur nachreagieren und hydrolysiert die Reaktionsmischung dann unter Rühren in ca. 1,5 1 Eiswasser. Die organische Phase wird abgetrennt, die wäßrige Phase nochmals mit Methylenchlorid extrahiert und beide organischen Phasen vereinigt. Diese werden neutral gewaschen, getrocknet und im Vakuum eingedampft. Das verbleibende gelbe Öl wird durch Verreiben mit Diisopropylether zur Kristallisation gebracht und das feste Produkt aus Diisopropylether umkristallisiert. Man erhält weiße Kristalle mit einem Schmelzpunkt von 87-89°C Ausbeute: 12,7 g (43% der Thorie).

| $C_{15}H_{19}ClO_2$ (266,77) | | | |
|---|---|---|---|
| Ber.: | C 67,53 | H 7,18 | Cl 13,29 |
| Gef.: | 67,68 | 7,29 | 13,11 |

Beispiel V

cis/trans-O-(4-Chlorphenylacetyl)-4-(4-N-[tert.-butoxycarbonyl]-methylaminomethyl)phenyl-cyclohexanol

a) 4-(4-Methylaminomethyl)phenyl-4-hydroxycyclohexanonethylenketal

Eine Lösung von 94 g (0,47 Mol) 4-Brom-(N-methyl)-benzylamin in 460 ml trockenem Tetrahydrofuran wird unter Stickstoffatmosphäre bei -30 bis -25°C zunächst mit 300 ml (0,48 Mol) einer 1,6-molaren Lösung von n-Butyllithium in Hexan und anschließend mit 52,5 g (0,48 Mol) Trimethylchlorsilan versetzt. Die Reaktionsmischung wird noch 15 Minuten bei dieser Temperatur gerührt und dann auf -75°C abgekühlt. Anschließend werden weitere 320 ml (0,51 Mol) einer 1,6-molaren Lösung von n-Butyllithium in Hexan so zugegeben, daß.die Temperatur -70°C nicht übersteigt. Die Mischung wird weitere 20 Minuten bei -75°C gerührt und dann innerhalb von 20 Minuten mit einer Lösung von 76 g (0,47 Mol) 1,4-Cyclohexandion-monoethylenketal in 200 ml Tetrahydrofuran versetzt, wobei die Temperatur -65°C nicht übersteigen darf. Anschließend wird die Reaktionsmischung zunächst 30 Minuten bei -70°C und danach ohne Außenkühlung gerührt, bis eine Temperatur von +20°C erreicht ist. Sie wird dann in eiskalter wäßriger Ammoniumchlorid-Lösung zersetzt und mehrmals mit Methylenchlorid extrahiert. Die vereinigten organischen Extrakte werden mit Natriumsulfat getrocknet, das Lösungsmittel wird entfernt und der verbleibende Rückstand wird aus Diisopropylether umkristallisiert. Es werden 77 g (59% der Theorie) 4-(4-Methylaminomethyl) phenyl-4-hydroxycyclohexanon-ethylenketal vom Schmelzpunkt 95-97°C erhalten.

b) 1-(4-Methylaminomethyl)phenyl-4-ethylendioxy-1-cyclohexen

Eine Mischung aus 68 g (0,24 Mol) 4-(4-Methylaminomethyl)phenyl-4-hydroxycyclohexanon-ethylenketal, 51 g (0,27 Mol) p-Toluolsulfonsäure-Monohydrat, 150 ml Ethylenglykol und 900 ml Toluol wird unter Rühren 2,5 Stunden zum Rückfluß erhitzt und das entstehende Reaktionswasser kontinuierlich entfernt. Die abgekühlte Reaktionsmischung wird mit ln-Natronlauge alkalisch gestellt (pH 12-13), die organische Schicht abgetrennt und die wäßrige Phase mehrmals mit Ethylacetat extrahiert. Die vereinigten organischen Phasen werden mit Natriumsulfat getrocknet und im Vakuum eingeengt. Es werden 63 g (ca. 100% der Theorie) 1-(4-Methylaminomethyl)-phenyl-4-ethylendioxy-l-cyclohexen als gelbliches Öl erhalten.

c) 1-(4-N-[tert.-Butoxycarbonyl]-methylaminomethyl)phenyl-4-ethylendioxy-1-cyclohexen

Eine Lösung von 63 g (0,24 Mol) des rohen 1-(4-Methylaminomethyl)phenyl-4-ethylendioxy-1-cyclohexen in 350 ml absolutem Tetrahydrofuran wird unter Rühren mit einer Lösung von 58 g (0,26 Mol) Di-tert.-Butyldicarbonat in 100 ml absolutem Tetrahydrofuran versetzt, wobei die Temperatur unter Kühlung zwischen 15-20°C gehalten wird. Nach Abklingen der $CO_2$-Entwicklung wird noch 10 Stunden bei Raumtemperatur belassen, das Lösungsmittel im Vakuum abdestilliert, der Rückstand mit Wasser versetzt und mehrfach mit Ether extrahiert. Nach dem Trocknen mit Natriumsulfat und Eindampfen werden 84 g (ca. 100% der Theorie) 1-(4-N-[tert.-Butoxycarbonyl]-methylaminomethyl)phenyl-4-ethylendioxy-1-cyclohexen als gelbliches Öl erhalten.

d) 4-(4-N-[tert.-Butoxycarbonyl]-methylaminomethyl)phenylcyclohexanon

Eine Lösung von 84 g (0,24 Mol) des rohen 1-(4-N-[tert.-Butoxycarbonyl]-methylaminomethyl)phenyl-4-ethylendioxy-1-cyclohexen in Methanol/Ethylacetat (250+250 ml) wird mit 10 g Palladium/Bariumsulfat-Katalysator versetzt und 4 Stunden bei Raumtemperatur und einem Wasserstoffdruck von 3 bar hydriert. Es wird vom Katalysator abgetrennt, das Lösungsmittel im Vakuum abdestilliert, der ölige Rückstand in Aceton/ Wasser (1400+140 ml) gelöst, und nach Zugabe von 8,5 g (0,034 Mol) Pyridiniumtosylat 15 Stunden zum Rückfluß erhitzt. Anschließend wird das Lösungsmittel im Vakuum abdestilliert, der Rückstand mit Wasser versetzt und mehrmals mit Methylenchlorid extrahiert. Nach dem Trocknen der organischen Phase mit Natriumsulfat und Eindampfen werden 61 g (77% der Theorie) 4-(4-N-[tert.-Butoxycarbonyl]-methylaminomethyl)phenyl-cyclohexanon als blaßgelbes Öl erhalten, das nach längerem Stehenlassen erstarrt.
Schmelzpunkt: 55-57°C.

e) 4-(4-N-[tert.-Butoxycarbonyl]-methylaminomethyl)phenylcyclohexanol (cis/trans-Gemisch)

Zu einer auf -10°C gekühlten Lösung von 11 g (0,035 Mol) 4-(4-N-[tert.-Butoxycarbonyl]-methylaminomethyl) phenyl-cyclohexanon in 70 ml absolutem Methanol wird unter Rühren portionsweise 1,31 g (0,035 Mol) Natriumborhydrid gegeben. Man läßt die Reaktionsmischung 0,5 Stunden bei -10°C und 2 Stunden bei Raumtemperatur nachreagieren und engt anschließend im Vakuum ein. Der verbleibende Rückstand wird mit Wasser versetzt und 1 Stunde bei Raumtemperatur gerührt. Das dabei entstehende feste Produkt wird abgesaugt, in Ethylacetat gelöst und diese Lösung über Natriumsulfat getrocknet. Nach Einengen im Vakuum verbleiben 8,6 g (77% der Theorie) eines Gemisches der cis- und trans-Form von 4-(4-N-[tert.-Butoxycarbonyl]-methylaminomethyl)phenyl-cyclohexanol als farbloses Öl. Dieses läßt sich säulenchromatographisch in die reinen Isomeren trennen (Aluminiumoxid neutral, Aktivitätsstuffe III, ICN; Petrolether/Ethylacetat = 3:1). $R_f$-Wert (Aluminiumoxid; Petrolether/Ethylacetat = 3:1): 0,21 (trans) und 0,31 (cis ).

f) O-(4-Chlorphenylacetyl)-4-(4-N-[tert.-butoxycarbonyl]-methylaminomethyl)phenyl-cyclohexanol (cis/trans-Gemisch)

Ein Gemisch aus 0,54 g (0,0032 Mol) 4-Chlorphenylessigsäure, 0,52 g (0,0032 Mol) N,N'-Carbonyldiimidazol und 20 ml Xylol wird unter Rühren 1 Stunde auf 60°C erhitzt. Anschließend wird eine Lösung von 0,85 g (0,0027 Mol) 4-(4-N-[tert.-Butoxycarbonyl]-methylaminomethyl)phenyl-cycohexanol (cis/trans-Gemisch) in 10 ml Xylol zugegeben und die Reaktionsmischung weitere 8 Stunden auf 160°C erhitzt. Nach dem Abkühlen wird im Vakuum eingeengt, der Rückstand mit Wasser versetzt und mit Ethylacetat extrahiert. Die organische Phase wird über Natriumsulfat getrocknet und im Vakuum eingedampft. Es verbleiben 1,3 g (ca. 100% der Theorie) O-(4-Chlorphenylacetyl)-4-(4-N-[tert.-butoxycarbonyl]-methylaminomethyl)-phenyl-cyclohexanol (cis/trans-Gemisch) als rotbraunes Öl. $R_f$-Wert (Aluminiumoxid; Petrolether/Ethylacetat = 3:1): 0,78 (trans) und 0,85 (cis).

Auf analoge Weise wurde die folgende Substanz synthetisiert:

(1) trans-O-(4-Chlorphenylacetyl)-4-(4-N-[tert.-butoxycarbonyl]-methylaminomethyl)phenyl-cyclohexanol
aus trans-4-(4-N-[tert.-Butoxycarbonyl]-methylaminomethyl)phenyl-cyclohexanol und 4-Chlorphenylessigsäure/N,N'-Carbonyldiimidazol.
Weiße Kristalle.
Schmelzpunkt: 94-96°C.

<u>Herstellung der Endprodukte:</u>

<u>Beispiel 1</u>

<u>trans-O-(4-Chlorbenzoyl)-4-(4-dimethylaminomethylphenyl)cyclohexanol</u>

Eine Lösung von 1,0 g (0,0043 Mol) trans-4-(4-Dimethylaminomethylphenyl)-cyclohexanol und 0,6 ml Triethylamin in 50 ml Methylenchlorid wird unter Rühren tropfenweise mit 0,75 g (0,0043 Mol) 4-Chlorbenzoylchlorid versetzt und 3 Stunden zum Rückfluß erhitzt. Nach dem Abkühlen wird mit 50 ml Wasser versetzt, mit Natronlauge auf pH 12-13 gestellt, die Methylenchloridphase abgetrennt und die wäßrige Phase nochmals mit Methylenchlorid extrahiert. Die vereinigten organischen Phasen werden über Natriumsulfat getrocknet und im Vakuum eingeengt. Der feste Rückstand wird säulenchromatographisch gereinigt (Aluminiumoxid neutral, Aktivitätsstufe III, ICN; Petrolether/Ethylacetat = 40: 1) Man erhält weiße Kristalle mit einem Schmelzpunkt von 94-95°C.
Ausbeute: 1,1 g (69% der Theorie)
$^1$H-NMR-Spektrum (200 MHz, $CDCl_3$); Signale bei ppm:
1,55-1,8 (m,4H); 1,9-2,1 (m,2H); 2,15-2,3 (s+m,6+2H); 2,5-2,7 (m,1H); 3,4 (s,2H); 4,9-5,1 (m,1H); 7,15-7,3 (m,4H); 7,4 (d,2H); 8,0 (d,2H).

Auf analoge Weise wurden die folgenden Substanzen synthetisiert:

(1) trans-O-Acetyl-4-(4-dimethylaminomethylphenyl)-cyclohexanol
aus trans-4-(4-Dimethylaminomethylphenyl)-cyclohexanol und Acetylchlorid/Triethylamin.
Farbloser Sirup.
$^1$H-NMR-Spektrum (200 MHz, $CDCl_3$); Signale bei ppm:
1,45-1,7 (m,4H); 1,9-2,05 (m,2H); 2,05-2,15 (s+m,3+2H); 2,23 (s,6H); 2,4-2,65 (m,1H); 3,4 (s,2H); 4,7-4,9 (m,1H); 7,1-7,3 (m,4H).

(2) trans-O-Butyryl-4-(4-dimethylaminomethylphenyl)-cyclohexanol
aus trans-4-(4-Dimethylaminomethylphenyl)-cyclohexanol und Buttersäurechlorid/Triethylamin.
Farbloses Öl.
$^1$H-NMR-Spektrum (200 MHz, $CDCl_3$); Signale bei ppm:
0,9-1,02 (t,3H); 1,45-1,75 (m,6H); 1,89-2,05 (m,2H); 2,05-2,18 (m,2H); 2,18-2,38 (s+t,6+2H); 2,4-2,6 (m,1H); 3,4 (s,2H); 4,7-4,9 (m,1H); 7,1-7,3 (m,4H).

(3) trans-O-Cyclopropanoyl-4-(4-dimethylaminomethylphenyl)-cyclohexanol
aus trans-4-(4-Dimethylaminomethylphenyl)-cyclohexanol und Cyclopropancarbonsäurechlorid/Triethylamin.
Farbloses Wachs.
$^1$H-NMR-Spektrum (200 MHz, $CDCl_3$); Signale bei ppm:
0,81-0,87 (m,2H); 0,95-1,02 (m,2H); 1,45-1,7 (m,4H); 1,9-2,0 (m,2H); 2,05-2,15 (m,2H), 2,24 (s,6H); 2,4-2,63 (2m, 2H); 3,4 (s,2H); 4,73-4,83 (m,1H); 7,12-7,25 (m,4H).

(4) trans-O-Cyclohexanoyl-4-(4-dimethylaminomethylphenyl)-cyclohexanol
aus trans-4-(4-Dimethylaminomethylphenyl)-cyclohexanol und Cyclohexancarbonsäurechlorid/Triethylamin.
Weiße Kristalle.
Schmelzpunkt: 66-68°C.

(5) cis-O-(4-Chlorphenylacetyl)-4-(4-dimethylaminomethylphenyl)-cyclohexanol
aus cis-4-(4-Dimethylaminomethylphenyl)-cyclohexanol und 4-Chlorphenylacetylchlorid/Triethylamin.
Farbloses Öl.
$^1$H-NMR-Spektrum (200 MHz, $CDCl_3$); Signale bei ppm:
1,5-1,75 (m,6H); 1,88-2,05 (m,2H); 2,25 (s,6H); 2,4-2,65 (m,1H); 3,4 (s,2H); 3,65 (s,2H); 5,05-5,15 (m,1H); 7,08 (d,2H); 7,2-7,4 (m,6H).

(6) trans-O-(4-Phenyl-3-butenoyl)-4-(4-dimethylaminomethylphenyl)-cyclohexanol
aus trans-4-(4-Dimethylaminomethylphenyl)-cyclohexanol und 4-Phenyl-3-butensäurechlorid/Triethylamin.
Weiße Kristalle.
Schmelzpunkt: 90-91°C.

(7) cis-O-(4-Phenyl-3-butenoyl)-4-(4-dimethylaminomethylphenyl)-cyclohexanol
aus cis-4-(4-Dimethylaminomethylphenyl)-cyclohexanol und 4-Phenyl-3-butensäurechlorid/Triethylamin.
Weiße Kristalle.
Schmelzpunkt: 71-73°C.

Beispiel 2

trans-O-(4-Chlorphenylacetyl)-4-(4-dimethylaminomethylphenyl)-cyclohexanol

Zu einem Gemisch aus 0,43 g (0,0025 Mol) 4-Chlorphenylessigsäure und 30 ml Xylol werden 0,41 g (0,0025 Mol) N,N'-Carbonyldiimidazol gegeben, wobei unter $CO_2$-Entwicklung ein weißes Produkt entsteht. Die Reaktionsmischung wird 1 Stunde unter Rühren auf 60°C erhitzt und dann 0,5 g (0,0021 Mol) trans-4-(4-Dimethylaminomethylphenyl)-cyclohexanol zugegeben. Es wird 12 Stunden unter Rühren auf 160°C erhitzt, auf Raumtemperatur abgekühlt, mit Wasser versetzt und mit 2N Natronlauge auf pH 12-13 gestellt. Die Xylolphase wird abgetrennt, die wäßrige Phase mehrmals mit Ethylacetat extrahiert, die organischen Phasen vereinigt, getrocknet und im Vakuum eingeengt. Der feste Rückstand wird säulenchromatographisch gereinigt (Aluminiumoxid basisch, Aktivitätsstufe III, ICN; Petrolether/ Ethylacetat = 10:1). Man erhält weiße Kristalle mit einem Schmelzpunkt von 75-77°C.
Ausbeute: 0,7 g (86% der Theorie).

$^1$H-NMR-Spektrum (200 MHz, $CDCl_3$); Signale bei ppm:
1,4-1,7 (m,4H); 1,8-2,15 (m,4H); 2,25 (s,6H); 2,4-2,6 (m,1H); 3,38 (s,2H); 3,6 (s,2H); 4,7-4,9 (m,1H); 7,1-7,35 (m,8H).

Auf analoge Weise wurden die folgenden Substanzen synthetisiert:

(1) trans-O-(5-Methylhexanoyl)-4-(4-dimethylaminomethylphenyl)-cyclohexanol
aus trans-4-(4-Dimethylaminomethylphenyl)-cyclohexanol und 5-Methylhexansäure/N,N'-Carbonyldiimidazol.
Weiße Kristalle.
Schmelzpunkt: 35-36°C.

(2) cis-O-(5-Methylhexanoyl)-4-(4-dimethylaminomethylphenyl)-cyclohexanol
aus cis-4-(4-Dimethylaminomethylphenyl)-cyclohexanol und 5-Methylhexansäure/N,N'-Carbonyldiimidazol.
Farbloses Ö1.
$^1$H-NMR-Spektrum (200 MHz, $CDCl_3$); Signale bei ppm:
0,9 (d,6H); 1,15-1,32 (m,2H); 1,5-1,88 (m,9H); 1,95-2,1 (m,2H); 2,25 (s,6H); 2,3 (d,2H); 2,48-2,69 (m,1H); 3,4 (s, 2H); 5,08-5,18 (m,1H); 7,12-7,3 (m,4H).

(3) trans-O-Cyclohexylacetyl-4-(4-dimethylaminomethylphenyl)-cyclohexanol
aus trans-4-(4-Dimethylaminomethylphenyl)-cyclohexanol und Cyclohexylessigsäure/N,N'-Carbonyldiimidazol.
Weiße Kristalle.
Schmelzpunkt: 37-39°C.

(4) trans-O-(2-Butenoyl)-4-(4-dimethylaminomethylphenyl)-cyclohexanol
aus trans-4-(4-Dimethylaminomethylphenyl)-cyclohexanol und Crotonsäure/N,N'-Carbonyldiimidazol.
Weiße Kristalle.
Schmelzpunkt: 69-71°C.

(5) trans-O-(2-Hexenoyl)-4-(4-dimethylaminomethylphenyl)-cyclohexanol
aus trans-4-(4-Dimethylaminomethylphenyl)-cyclohexanol und 2-Hexensäure/N,N'-Carbonyldiimidazol.
Weiße Kristalle.
Schmelzpunkt: 40-42°C.

(6) trans-O-(3-Cyclohexylpropenoyl)-4-(4-dimethylaminomethylphenyl)-cyclohexanol
aus trans-4-(4-Dimethylaminomethylphenyl)-cyclohexanol und 3-Cyclohexylpropensäure/N,N'-Carbonyldiimidazol.
Weiße Kristalle.
Schmelzpunkt: 46-47°C.

(7) trans-O-Benzoyl-4-(4-dimethylaminomethylphenyl)-cyclohexanol
aus trans-4-(4-Dimethylaminomethylphenyl)-cyclohexanol und Benzoesäure/N,N'-Carbonyldiimidazol.
Weiße Kristalle.
Schmelzpunkt: 68-70°C.

(8) trans-O-(4-Chlor-3-methylbenzoyl)-4-(4-dimethylaminomethylphenyl)-cyclohexanol
aus trans-4-(4-Dimethylaminomethylphenyl)-cyclohexanol und 4-Chlor-3-methylbenzoesäure/N,N'-Carbonyldiimidazol.
Weiße Kristalle.
Schmelzpunkt: 100-102°C.

(9) trans-O-(2-Naphthoyl)-4-(4-dimethylaminomethylphenyl)-cyclohexanol
aus trans-4-(4-Dimethylaminomethylphenyl)-cyclohexanol und 4-Naphthoesäure/N,N'-Carbonyldiimidazol.
Weiße Kristalle.
Schmelzpunkt: 110-112°C.

(10) trans-O-Phenylacetyl-4-(4-dimethylaminomethylphenyl)-cyclohexanol
aus trans-4-(4-Dimethylaminomethylphenyl)-cyclohexanol und Phenylessigsäure/N,N'-Carbonyldiimidazol.
Weiße Kristalle.
Schmelzpunkt: 38-40°C.

(11) trans-O-(4-Fluorphenylacetyl)-4-(4-dimethylaminomethylphenyl)-cyclohexanol
aus trans-4-(4-Dimethylaminomethylphenyl)-cyclohexanol und 4-Fluorphenylessigsäure/N,N'-Carbonyldiimidazol.
Weiße Kristalle.
Schmelzpunkt: 68-70°C.

(12) cis-O-(4-Fluorphenylacetyl)-4-(4-dimethylaminomethylphenyl)-cyclohexanol
aus cis-4-(4-Dimethylaminomethylphenyl)-cyclohexanol und 4-Fluorphenylessigsäure/N,N'-Carbonyldiimidazol.
Farbloses Öl.
$^1$H-NMR-Spektrum (200 MHz, $CDCl_3$); Signale bei ppm:
1,5-1,75 (m,6H); 1,85-2,05 (m,2H); 2,3 (s,6H); 2,4-2,65 (m,1H); 3,43 (s,2H); 3,65 (s,2H); 5,05-5,15 (m,1H); 7,0-7,15 (m,4H); 7,2-7,38 (m,4H).

(13) trans-O-(4-Bromphenylacetyl)-4-(4-dimethylaminomethylphenyl)-cyclohexanol
aus trans-4-(4-Dimethylaminomethylphenyl)-cyclohexanol und 4-Bromphenylessigsäure/N,N'-Carbonyldiimidazol.
Weiße Kristalle.
Schmelzpunkt: 72-74°C.

(14) trans-O-(3,4-Dichlorphenylacetyl)-4-(4-dimethylaminomethylphenyl)-cyclohexanol
aus trans-4-(4-Dimethylaminomethylphenyl)-cyclohexanol und 3,4-Dichlorphenylessigsäure/N,N'-Carbonyldiimidazol.
Weiße Kristalle.
Schmelzpunkt: 95-97°C.

(15) cis-O-(3,4-Dichlorphenylacetyl)-4-(4-dimethylaminomethylphenyl)-cyclohexanol
aus cis-4-(4-Dimethylaminomethylphenyl)-cyclohexanol und 3,4-Dichlorphenylessigsäure/N,N'-Carbonyldiimidazol.
Farbloses Öl.
$^1$H-NMR-Spektrum (200 MHz, $CDCl_3$); Signale bei ppm:
1,5-1,75 (m,6H); 1,9-2,05 (m,2H); 2,28 (s,6H); 2,4-2,65 (m,1H); 3,4 (s,2H); 3,62 (s,2H); 5,8-5,17 (m,1H); 7,05-7,3 (m,5H); 7,35-7,49 (m,2H).

(16) trans-O-(2,4-Dichlorphenylacetyl)-4-(4-dimethylaminomethylphenyl)-cyclohexanol
aus trans-4-(4-Dimethylaminomethylphenyl)-cyclohexanol und 2,4-Dichlorphenylessigsäure/N,N'-Carbonyldiimidazol.
Weiße Kristalle.
Schmelzpunkt: 78-80°C.

(17) trans-O-(p-Tolylacetyl)-4-(4-dimethylaminomethylphenyl)-cyclohexanol
aus trans-4-(4-Dimethylaminomethylphenyl)-cyclohexanol und p-Tolylessigsäure/N,N'-Carbonyldiimidazol.
Weiße Kristalle.
Schmelzpunkt: 40-42°C.

(18) trans-O-(4-[Trifluormethyl]-phenylacetyl)-4-(4-dimethylaminomethylphenyl)-cyclohexanol
aus -rans-4-(4-Dimethylaminomethylphenyl)-cyclohexanol und 4-(Trifluormethyl)-phenylessigsäure/N,N'-Carbonyldiimidazol.
Weiße Kristalle.
Schmelzpunkt: 73-75°C.

(19) trans-O-(4-Methoxyphenylacetyl)-4-(4-dimethylaminomethylphenyl)-cyclohexanol
aus trans-4-(4-Dimethylaminomethylphenyl)-cyclohexanol und 4-Methoxyphenylessigsäure/N,N'-Carbonyldiimidazol.
Weiße Kristalle.
Schmelzpunkt: 47-49°C.

(20) trans-O-(4-Nitrophenylacetyl)-4-(4-dimethylaminomethylphenyl)-cyclohexanol
aus trans-4-(4-Dimethylaminomethylphenyl)-cyclohexanol und 4-Nitrophenylessigsäure/N,N'-Carbonyldiimidazol.

Gelbliche Kristalle.
Schmelzpunkt: 136-137°C.

(21) trans-O-[3-(4-Fluorphenyl)-propionyl]-4-(4-dimethylaminomethylphenyl)-cyclohexanol
aus trans-4-(4-Dimethylaminomethylphenyl)-cyclohexanol und 3-(4-Fluorphenyl)-propionsäure/N,N'-Carbonyldiimidazol.
Weiße Kristalle.
Schmelzpunkt: 58-59°C.

(22) trans-O-[3-(4-Chlorphenyl)-propionyl]-4-(4-dimethylaminomethylphenyl)-cyclohexanol
aus trans-4-(4-Dimethylaminomethylphenyl)-cyclohexanol und 3-(4-Chlorphenyl)-propionsäure/N,N'-Carbonyldiimidazol.
Weiße Kristalle.
Schmelzpunkt: 85-87°C.

(23) trans-O-(4-Biphenylacetyl)-4-(4-dimethylaminomethylphenyl)-cyclohexanol
aus trans-4-(4-Dimethylaminomethylphenyl)-cyclohexanol und 4-Biphenylessigsäure/N,N'-Carbonyldiimidazol.
Weiße Kristalle.
Schmelzpunkt: 88-89°C.

(24) trans-O-(2-Naphthylacetyl)-4-(4-dimethylaminomethylphenyl)-cyclohexanol
aus trans-4-(4-Dimethylaminomethylphenyl)-cyclohexanol und 2-Naphthylessigsäure/N,N'-Carbonyldiimidazol.
Weiße Kristalle.
Schmelzpunkt: 85-87°C.

(25) trans-O-[2-(1,2,3,4-Tetrahydro)naphthoyl]-4-(4-dimethylaminomethylphenyl)-cyclohexanol
aus trans-4-(4-Dimethylaminomethylphenyl)-cyclohexanol und 2-(1,2,3,4-Tetrahydro)naphthoesäure/N,N'-Carbonyldiimidazol.
Weiße Kristalle.
Schmelzpunkt: 95-96°C.

(26) cis-O-[2-(1,2,3,4-Tetrahydro)naphthoyl]-4-(4-dimethylaminomethylphenyl)-cyclohexanol
aus cis-4-(4-Dimethylaminomethylphenyl)-cyclohexanol und 2-(1,2,3,4-Tetrahydro)naphthoesäure/N,N'-Carbonyldiimidazol.
Farbloses Öl.
$^1$H-NMR-Spektrum (200 MHz, $CDCl_3$); Signale bei ppm:
1,55-1,85 (m,6H); 1,9-2,1 (m,3H); 2,15-2,32 (s+m,6+1H); 2,4-2,65 (m,1H); 2,72-2,95 (m,3H); 3,05 (d,2H); 3,4 (s, 2H); 5,1-5,2 (m,1H); 7,05-7,3 (2m,8H).

(27) trans-O-(2-Phenylpropionyl)-4-(4-dimethylaminomethylphenyl)-cyclohexanol
aus trans-4-(4-Dimethylaminomethylphenyl)-cyclohexanol und 2-Phenylpropionsäure/N,N'-Carbonyldiimidazol.
Farbloses Wachs.
$^1$H-NMR-Spektrum (200 MHz, $CDCl_3$); Signale bei ppm:
1,25-1,7 (d+m,3+3H); 1,8-2,15 (m,5H); 2,3 (s,6H); 2,38-2,6 (m,1H); 3,4 (s,2H); 3,7 (q,1H); 4,68-4,9 (m,1H); 7,15 (d,2H); 7,18-7,38 (d+m,2+5H).

(28) cis-O-(2-Phenylpropionyl)-4-(4-dimethylaminomethylphenyl)-cyclohexanol
aus cis-4-(4-Dimethylaminomethylphenyl)-cyclohexanol und 2-Phenylpropionsäure/N,N'-Carbonyldiimidazol.
Farbloses Öl.
$^1$H-NMR-Spektrum (200 MHz, $CDCl_3$); Signale bei ppm:
1,3-1,7 (d+m,3+6H); 1,8-2,05 (m,2H); 2,25 (s,6H); 2,35-2,58 (m,1H); 3,4 (s,2H); 3,78 (q,1H); 5,0-5,1(m,1H); 7,0 (d,2H); 7,2 (d,2H); 7,25-7,4 (m,5H).

(29) trans-O-(4-Fluorcinnamoyl)-4-(4-dimethylaminomethylphenyl)-cyclohexanol
aus trans-4-(4-Dimethylaminomethylphenyl)-cyclohexanol und 4-Fluorzimtsäure/N,N'-Carbonyldiimidazol.
Weiße Kristalle.
Schmelzpunkt: 118-120°C.

(30) cis-O-(4-Fluorcinnamoyl)-4-(4-dimethylaminomethylphenyl)-cyclohexanol
aus cis-4-(4-Dimethylaminomethylphenyl)-cyclohexanol und 4-Fluorzimtsäure/N,N'-Carbonyldiimidazol.
Weiße Kristalle.
Schmelzpunkt: 66-68°C.

(31) trans-O-(4-Chlorcinnamoyl)-4-(4-dimethylaminomethylphenyl)-cyclohexanol
aus trans-4-(4-Dimethylaminomethylphenyl)-cyclohexanol und 4-Chlorzimtsäure/N,N'-Carbonyldiimidazol.
Weiße Kristalle.
Schmelzpunkt: 131-133°C.

(32) cis-O-(4-Chlorcinnamoyl)-4-(4-dimethylaminomethylphenyl)-cyclohexanol
aus cis-4-(4-Dimethylaminomethylphenyl)-cyclohexanol und 4-Chlorzimtsäure/N,N'-Carbonyldiimidazol.
Weiße Kristalle.
Schmelzpunkt: 88-90°C.

(33) trans-O-(4-[Trifluormethyl]-cinnamoyl)-4-(4-dimethylaminomethylphenyl)-cyclohexanol
aus trans-4-(4-Dimethylaminomethylphenyl)-cyclohexanol und 4-(Trifluormethyl)-zimtsäure/N,N'-Carbonyldiimidazol.
Weiße Kristalle.
Schmelzpunkt: 134-136°C.

(34) cis-O-(4-[Trifluormethyl]-cinnamoyl)-4-(4-dimethylaminomethylphenyl)-cyclohexanol
aus cis-4-(4-Dimethylaminomethylphenyl)-cyclohexanol und 4-(Trifluormethyl)-zimtsäure/N,N'-Carbonyldiimidazol.
Weiße Kristalle.
Schmelzpunkt: 61-63°C.

(35) trans-O-(5-Chlor-2-thenoyl)-4-(4-dimethylaminomethylphenyl)-cyclohexanol
aus trans-4-(4-Dimethylaminomethylphenyl)-cyclohexanol und 5-Chlor-2-thiophencarbonsäure/N,N'-Carbonyldiimidazol.
Weiße Kristalle.
Schmelzpunkt: 95-97°C.

(36) trans-O-Nicotinoyl-4-(4-dimethylaminomethylphenyl)cyclohexanol
aus trans-4-(4-Dimethylaminomethylphenyl)-cyclohexanol und Nicotinsäure/N,N'-Carbonyldiimidazol.
Weiße Kristalle.
Schmelzpunkt: 86-88°C.

(37) trans-O-(2-Furoyl)-4-(4-dimethylaminomethylphenyl)cyclohexanol
aus trans-4-(4-Dimethylaminomethylphenyl)-cyclohexanol und 2-Furancarbonsäure/N,N'-Carbonyldiimidazol.
Weiße Kristalle.
Schmelzpunkt: 58-60°C.

(38) trans-O-(3,4-Dimethoxyphenylacetyl)-4-(4-dimethylaminomethylphenyl)-cyclohexanol
aus trans-4-(4-Dimethylaminomethylphenyl)-cyclohexanol und 3,4-Dimethoxyphenylessigsäure/N,N'-Carbonyldiimidazol.
Weiße Kristalle.
Schmelzpunkt: 32-34°C.

(39) trans-O-(4-Amino-3-chlorphenylacetyl)-4-(4-dimethylaminomethylphenyl)-cyclohexanol
aus trans-4-(4-Dimethylaminomethylphenyl)-cyclohexanol und 4-Amino-3-chlorphenylessigsäure/N,N'-Carbonyldiimidazol.
Weiße Kristalle.
Schmelzpunkt: 83-85°C.

(40) trans-O-(4-Amino-3,5-dichlorphenylacetyl)-4-(4-dimethylaminomethylphenyl)-cyclohexanol
aus trans-4-(4-Dimethylaminomethylphenyl)-cyclohexanol und 4-Amino-3,5-dichlorphenylessigsäure/N,N'-Carbonyldiimidazol.

Weiße Kristalle.
Schmelzpunkt: 78-80°C.

(41) trans-O-(4-Chlorphenylacetyl)-4-(4-diethylaminomethylphenyl)-cyclohexanol
aus trans-4-(4-Diethylaminomethylphenyl)-cyclohexanol und 4-Chlorphenylessigsäure/N,N'-Carbonyldiimidazol.
Farbloses Öl.
$^1$H-NMR-Spektrum (200 MHz, $CDCl_3$); Signale bei ppm: 1,05 (t,6H); 1,4-1,72 (m,4H); 1,9-2,2 (m,4H); 2,4-2,6 (q+m, 5H); 3,5 (s,2H); 3,6 (s,2H); 4,7-4,9 (m,1H); 7,12 (d,2H); 7,18-7,35 (m,6H).

(42) trans-O-(4-Chlorphenylacetyl)-4-(4-dipropylaminomethylphenyl)-cyclohexanol
aus trans-4-(4-Dipropylaminomethylphenyl)-cyclohexanol und 4-Chlorphenylessigsäure/N,N'-Carbonyldiimidazol.
Farbloses Öl.
$^1$H-NMR-Spektrum (200 MHz, $CDCl_3$); Signale bei ppm:
0,88 (t,6H); 1,38-1,7 (m,8H); 1,9-2,19 (m,4H); 2,35 (q,4H); 2,4-2,6 (m,1H); 3,5 (s,2H); 3,6 (s,2H); 4,7-4,9 (m,1H); 7,1 (d,2H); 7,15-7,38 (m,6H).

(43) trans-O-(4-Chlorphenylacetyl)-4-(4-[N-methylbutylamino]methylphenyl)-cyclohexanol
aus trans-4-(4-[N-Methylbutylamino]-methylphenyl)-cyclohexanol und 4-Chlorphenylessigsäure/N,N'-Carbonyldiimidazol.
Farbloses Öl.
$^1$H-NMR-Spektrum (200 MHz, $CDCl_3$); Signale bei ppm:
0,9 (t,3H); 1,2-1,7 (m,8H); 1,8-2,15 (m,4H); 2,18 (s,3H); 2,35 (t,2H); 2,4-2,6 (m,1H); 3,41 (s,2H); 3,6 (s,2H); 4,7-4,9 (m,1H); 7,1 (d,2H); 7,2-7,35 (m,6H).

(44) trans-O-(4-Chlorphenylacetyl)-4-(4-diallylaminomethylphenyl)-cyclohexanol
aus trans-4-(4-Diallylaminomethylphenyl)-cyclohexanol und 4-Chlorphenylessigsäure/N,N'-Carbonyldiimidazol.
Farbloses Öl.
$^1$H-NMR-Spektrum (200 MHz, $CDCl_3$); Signale bei ppm:
1,4-1,75 (m,4H); 1,9-2,2 (m,4H); 2,4-2,6 (m,1H); 3,0-3,18 (dd,4H); 3,5 (s,2H); 3,6 (s,2H); 4,7-4,9 (m,1H); 5,1-5,3 (m,4H); 5,75-6,0 (m,2H); 7,12 (d,2H); 7,15-7,38 (m,6H).

(45) trans-O-(4-Chlorphenylacetyl)-4-(4-[N-pyrrolidino]-methylphenyl)-cyclohexanol
aus trans-4-(4-[N-Pyrrolidino]methylphenyl)-cyclohexanol und 4-Chlorphenylessigsäure/N,N'-Carbonyldiimidazol.
Weiße Kristalle.
Schmelzpunkt: 57-59°C.

(46) trans-O-(4-Chlorphenylacetyl)-4-(4-[N-piperidino]methylphenyl)-cyclohexanol
aus trans-4-(4-[N-Piperidino]methylphenyl)-cyclohexanol und 4-Chlorphenylessigsäure/N,N'-Carbonyldiimidazol.
Weiße Kristalle.
Schmelzpunkt: 87-89°C.

(47) trans-O-(4-Chlorphenylacetyl)-4-(4-[N-morpholino]methylphenyl)-cyclohexanol
aus trans-4-(4-[N-Morpholino]methylphenyl)-cyclohexanol und 4-Chlorphenylessigsäure/N,N'-Carbonyldiimidazol.
Weiße Kristalle.
Schmelzpunkt: 114-116°C.

(48) trans-O-(4-Chlorphenylacetyl)-4-(4-[N-methyl-N'-piperazino]methylphenyl)-cyclohexanol
aus trans-4-(4-[N-Methyl-N'-piperazino]methylphenyl)-cyclohexanol und 4-Chlorphenylessigsäure/N,N'-Carbonyldiimidazol.
Weiße Kristalle.
Schmelzpunkt: 97-99°C.

(49) trans-O-(3,4-[Methylendioxy]-phenylacetyl)-4-(4-dimethylaminomethylphenyl)-cyclohexanol
aus trans-4-(4-Dimethylaminomethylphenyl)-cyclohexanol und 3,4-(Methylendioxy)-phenylessigsäure/N,N'-Carbonyldiimidazol.
Farbloses Öl.
$^1$H-NMR-Spektrum (200 MHz, $CDCl_3$); Signale bei ppm:

1,4-1,7 (m,4H), 1,8-2,0 (m,2H), 2,0-2,15 (m,2H), 2,25 (s,6H), 2,4-2,6 (m,1H), 3,38 (s,2H), 3,5 (s,2H), 4,7-4,9 (m, 1H), 5,94 (s,2H), 6,7-6,85 (m,3H), 7,1-7,3 (m,4H).

Beispiel 3

cis-O-(4-Chlorbenzoyl)-4-(4-dimethylaminomethylphenyl)-cyclohexanol

0,24 g (0,001 mol) cis-4-(4-Dimethylaminomethylphenyl)-cyclohexanol, 0,34 ml (0,0025 Mol) Triethylamin und 0,12 g (0,001 Mol) Dimethylaminopyridin werden in 20 ml Methylenchlorid gelöst, mit 0,175 g (0,001 Mol) 4-Chlorbenzoylchlorid versetzt und 12 Stunden bei Raumtemperatur gerührt. Die Reaktionsmischung wird mit Wasser versetzt und mit Natronlauge auf pH 12-13 gestellt. Die Methylenchloridphase wird abgetrennt, die wäßrige Phase mehrmals mit Methylenchlorid extrahiert und die vereinigten organischen Phasen werden mit gesättigter Kochsalzlösung gewaschen, getrocknet und im Vakuum eingeengt. Der Rückstand wird säulenchromatographisch gereinigt (Aluminiumoxid neutral, Aktivitätsstufe III, ICN; Petrolether/Ethylacetat = 45:1)
Weiße Kristalle.
Schmelzpunkt: 96-97°C.
Ausbeute: 0,27 g (73% der Theorie)
$^1$H-NMR-Spektrum (200 MHz, $CDCl_3$); Signale bei ppm:
1,7-2,0 (m,6H); 2,1-2,25 (m,2H); 2,28 (s,6H); 2,55-2,75 (m,1H); 3,4 (s,2H); 5,33-5,4 (m,1H); 7,15-7,3 (m,4H); 7,45 (d, 2H); 8,2 (d,2H).

Beispiel 4

trans-O-Acetyl-4-(4-diethylaminomethylphenyl)-cyclohexanol

Eine Lösung von 1 g (3,75 mMol) trans-O-Acetyl-4-(4-chlormethylphenyl)-cyclohexanol in 10 ml Dimethylformamid wird mit 0,52 g (3,75 mMol) Kaliumcarbonat und 0,27 g (3,75 mMol) Diäthylamin versetzt. Diese Mischung wird unter Rühren 6 Stunden auf 50°C erwärmt, anschließend mit Wasser versetzt und mit Methylenchlorid extrahiert. Die organische Phase wird getrocknet, im Vakuum eingeengt und der verbleibende Rückstand wird säulenchromatographisch gereinigt (Aluminiumoxid basisch, Aktivitätsstufe III, ICN; Petrolether/Ethylacetat = 15:1).
Farbloses Öl.
Ausbeute: 0,79 g (69% der Theorie)
$^1$H-NMR-Spektrum (200 MHz, $CDCl_3$); Signale bei ppm:
1,05 (t,6H); 1,45-1,75 (m,4H); 1,9-2,2 (s+m,7H); 2,4-2,6 (q+m,5H); 3,55 (s,2H); 4,68-4,9 (m,1H); 7,12 (d,2H); 7,28 (d, 2H).

Auf analoge Weise wurden die folgenden Substanzen synthetisiert:

(1) trans-O-Acetyl-4-(4-dipropylaminomethylphenyl)-cyclohexanol
aus trans-O-Acetyl-4-(4-chlormethylphenyl)-cyclohexanol und Dipropylamin.
Farbloses Öl.
$^1$H-NMR-Spektrum (200 MHz, $CDCl_3$); Signale bei ppm:
0,9 (t,6H); 1,35-1,75 (m,8H); 1,9-2,2 (s+m,7H); 2,3-2,6 (q+m,5H); 3,5 (s,2H); 4,65-4,9 (m,1H); 7,1 (d,2H); 7,25 (d, 2H).

(2) trans-O-Acetyl-4-(4-[N-methylbutylamino]-methylphenyl)cyclohexanol
aus trans-O-Acetyl-4-(4-chlormethylphenyl)-cyclohexanol und N-Methylbutylamin.
Farbloses Öl.
$^1$H-NMR-Spektrum (200 MHz, $CDCl_3$); Signale bei ppm:
0,9 (t,3H); 1,2-1,75 (m,10H); 1,9-2,2 (2s+m,8H); 2,38 (t,2H); 2,4-2,6 (m,1H); 3,45 (s,2H); 4,7-4,9 (m,1H); 7,15 (d, 2H); 7,25 (d,2H).

(3) trans-O-Acetyl-4-(4-diallylaminomethylphenyl)-cyclohexanol
aus trans-O-Acetyl-4-(4-chlormethylphenyl)-cyclohexanol und Diallylamin.
Farbloses Öl.
$^1$H-NMR-Spektrum (200 MHz, $CDCl_3$); Signale bei ppm:
1,4-1,7 (m,4H), 1,9-2,18 (s+m,7H); 2,4-2,6 (m,1H); 3,09 (dd,4H); 3,52 (s,2H); 4,7-4,9 (m,1H); 5,01-5,3 (m,4H); 5,75-6,0 (m,2H); 7,12 (d,2H); 7,35 (d,2H).

(4) trans-O-Acetyl-4-(4-[N-pyrrolidino]methylphenyl)-cyclohexanol
aus trans-O-Acetyl-4-(4-chlormethylphenyl)-cyclohexanol und Pyrrolidin.
Farblose Kristalle.
Schmelzpunkt: 43-45°C.

(5) trans-O-Acetyl-4-(4-[N-morpholinolmethylphenyl)-cyclohexanol
aus trans-O-Acetyl-4-(4-chlormethylphenyl)-cyclohexanol und Morpholin.
Farblose Kristalle.
Schmelzpunkt: 53-55°C.

(6) trans-O-Acetyl-4-(4-[N-piperidino]methylphenyl)-cyclohexanol
aus trans-O-Acetyl-4-(4-chlormethylphenyl)-cyclohexanol und Piperidin.
Farblose Kristalle.
Schmelzpunkt: 62-64°C.

(7) trans-O-Acetyl-4-[N-Methyl-N'-piperazino]methylphenyl)cyclohexanol
aus trans-O-Acetyl-4-(4-chlormethylphenyl)-cyclohexanol und N-Methylpiperazin.
Farblose Kristalle.
Schmelzpunkt: 50-52°C.

Beispiel 5

trans-O-(4-Chlorphenylacetyl)-4-(4-methylaminomethylphenyl)cyclohexanol

Eine Lösung von 8,9 g (0,019 Mol) trans-O-(4-Chlorphenylacetyl)-4-(4-N-[tert.-butoxycarbonyl]-methylaminomethyl)-phenyl-cyclohexanol in 200 ml Methylenchlorid wird mit 35 ml Trifluoressigsäure versetzt und 2 Stunden bei Raumtemperatur gerührt. Anschließend werden die flüchtigen Anteile im Vakuum abdestilliert, der Rückstand in Methylenchlorid aufgenommen und mit gesättigter Natriumhydrogencarbonat-Lösung neutral gewaschen. Die organische Phase wird über Natriumsulfat getrocknet und im Vakuum eingeengt. Es verbleibt ein gelbliches Öl, das säulenchromatographisch gereinigt wird (Aluminiumoxid basisch, Aktivitätsstufe III, ICN; Petrolether/ Ethylacetat/Methanol = 10:10:1).
Man erhält gelblich-weiße Kristalle vom Schmelzpunkt 65-67°C.
Ausbeute: 6,4 g (91% der Theorie).
$^1$H-NMR-Spektrum (200 MHz, $CDCl_3$); Signale bei ppm:
1,4-1,7 (m,4H), 1,8-2,0 (m,2H), 2,0-2,2 (m,2H), 2,4-2,6 (s+m,3+1H), 3,58 (s,2H), 3,7 (s,2H), 4,7-4,9 (m,1H), 7,1-7,35 (m,8H).

Auf analoge Weise wurde die folgende Substanz synthetisiert:

(1) O-(4-Chlorphenylacetyl)-4-(4-methylaminomethylphenyl)-cyclohexanol (cis/trans-Gemisch)
aus O-(4-Chlorphenylacetyl)-4-(4-N-[tert.-butoxycarbonyl]-methylaminomethyl)phenyl-cyclohexanol (cis/trans-Gemisch) und Trifluoressigsäure.
Gelbes Öl.
$R_f$-Wert (Aluminiumoxid; Petrolether/Ethylacetat/Methanol = 10:10:1): 0,28-0,53.

Beispiel 6

O-(4-Chlorphenylacetyl)-4-(4-N-[carboxamidomethyl]-methylaminomethyl)phenyl-cyclohexanol (cis/trans-Gemisch)

Ein Gemisch aus 1,0 g (0,0027 Mol) O-(4-Chlorphenylacetyl)-4-(4-methylaminomethylphenyl)-cyclohexanol (cis/trans-Gemisch), 0,5 g (0,0027 Mol) Iodacetamid, 0,37 g (0,0027 Mol) Kaliumcarbonat und 5 ml Dimethylformamid wird unter Rühren 2 Stunden auf 50°C erwärmt. Nach Abkühlen auf Raumtemperatur wird mit Wasser versetzt und mit Ethylacetat extrahiert. Die organische Phase wird über Natriumsulfat getrocknet, die flüchtigen Anteile im Vakuum abdestilliert und der Rückstand säulenchromatographisch gereinigt (Aluminiumoxid basisch, Aktivitätsstufe III, ICN; Petrolether/Ethylacetat/Methanol = 60:40:2,5).
Man erhält ein weißes kristallines Produkt, das ab 110°C sintert und zwischen 128-132°C schmilzt.
$^1$H-NMR-Spektrum (200 MHz, $CDCl_3/CD_3OD$); Signale bei ppm:
1,4-1,75 (m,5H), 1,8-2,2 (m,3H), 2,32 (dd,3H); 2,4-2,63 (m,1H), 3,0 (dd,2H), 3,5-3,7 (dd+dd,2+2H), 4,7-4,9 (m,0,5H), 5,08-5,15 (m,0,5H), 7,0-7,4 (m,8H).

Auf analoge Weise wurden die folgenden Substanzen synthetisiert:

(1) trans-O-(4-Chlorphenylacetyl)-4-(4-N-[carbethoxymethyl]-methylaminomethyl)phenyl-cyclohexanol
aus trans-O-(4-Chlorphenylacetyl)-4-(4-methylaminomethylphenyl)-cyclohexanol, Bromessigsäureethylester und Kaliumcarbonat/Dimethylformamid.
Weißes Festprodukt.
Schmelzpunkt: 40-42°C.

(2) trans-O-(4-Chlorphenylacetyl)-4-(4-N-[3-hydroxypropyl]-methylaminomethyl)phenyl-cyclohexanol
aus trans-O-(4-(Chlorphenylacetyl)-4-(4-methylaminomethylphenyl)-cyclohexanol, 3-Brompropanol und Kaliumcarbonat/Dimethylformamid.
Weiße Kristalle.
Schmelzpunkt: 75-77°C.

Beispiel 7

trans-O-(4-Chlorphenylacetyl)-4-(4-dimethylaminomethylphenyl)-cyclohexanol-hydrochlorid

Eine Lösung von 0,39 g (0,001 Mol) trans-O-(4-Chlorphenylacetyl)-4-(4-dimethylaminomethylphenyl)-cyclohexanol in 10 ml Diethylether wird bei Raumtemperatur unter Rühren tropfenweise mit der 1,5-fachen equimolaren Menge Chlorwasserstoff in Isopropanol versetzt. Der ausgefallene Niederschlag wird 1 Stunde bei Raumtemperatur belassen, abgesaugt, wiederholt mit Diethylether gewaschen und getrocknet. Man erhält weiße Kristalle vom Schmelzpunkt 231-233°C.
Ausbeute: 0,32 g (76% der Theorie).

| $C_{23}H_{29}Cl_2NO_2$ (422,40) | | | | |
|---|---|---|---|---|
| Ber.: | C 65,40 | H 6,92 | N 3,32 | Cl 16,79 |
| Gef.: | 65,33 | 7,06 | 3,45 | 16,92 |

Beispiel 8

trans-O-(4-Chlorphenylacetyl)-4-(4-dimethylaminomethylphenyl)-cyclohexanol-tartrat

In 7 ml absolutem Ethanol werden zunächst 0,15 g (0,001 Mol) wasserfreie Weinsäure und dann 0,39 g (0,001 Mol) trans-O-(4-Chlorphenylacetyl)-4-(4-dimethylaminomethylphenyl)-cyclohexanol gelöst. Die klare Lösung wird daraufhin bis zur leichten Trübung mit Diethylether versetzt und 8 Stunden bei +4°C stehen gelassen. Das ausgefallene kristalline Produkt wird abgesaugt, mit Diethylether gewaschen und getrocknet. Schmelzpunkt: 169-171°C.
Ausbeute: 0,46 g (86% der Theorie).

| $C_{27}H_{34}ClNO_8$ (536,02) | | | | |
|---|---|---|---|---|
| Ber.: | C 60,50 | H 6,39 | N 2,61 | Cl 6,61 |
| Gef.: | 60,37 | 6,38 | 2,65 | 6,73 |

Im folgenden wird die Herstellung pharmazeutischer Anwendungsformen anhand einiger Beispiele beschrieben:

Beispiel I

Tabletten mit 5 mg trans-O-(4-Chlorphenylacetyl)-4-(4-dimethylaminomethyl-phenyl)-cyclohexanol

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragees mit 5 mg trans-O-(4-Chlorphenylacetyl)-4-(4-dimethylaminomethyl-phenyl)-cyclohexanol

Die nach Beispiel I hergestellten Tabletten werden nach bekanntem Verfahren mit einer Hülle überzogen, die im wesentlichen aus Zucker und Talkum besteht. Die fertigen Dragees werden mit Hilfe von Bienenwachs poliert. Dragéegewicht: 300 mg

Beispiel III

Suppositorien mit 5 mg trans-O-(4-Chlorphenylacetyl)-4-(4-dimethylaminomethyl-phenyl)-cyclohexanol

| Zusammensetzung: | |
|---|---|
| 1 Zäpfchen enthält: | |
| Wirkstoff | 5,0 mg |
| Zäpfchenmasse (z.B. Witepsol W 45$^{(R)}$) | 1 695,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus. Zäpfchengewicht 1,7 g

Beispiel IV

Kapseln mit 5 mg trans-O-(4-[Trifluormethyl]-phenylacetyl)-4-(4-dimethylaminomethyl-phenyl)-cyclohexanol

| Zusammensetzung: | |
|---|---|
| 1 Kapsel enthält: | |
| Wirksubstanz | 5,0 mg |
| Lactose | 82,0 mg |
| Stärke | 82,0 mg |
| Magnesiumstearat | 1,0 mg |
| | 170,0 mg |

Herstellungsverfahren:

Die Pulvermischung wird intensiv gemischt und auf einer Kapselabfüllmaschine in Hartgelatine-Steckkapseln der Größe 3 abgefüllt, wobei das Endgewicht laufend überprüft wird.

Beispiel V

Tabletten mit 5 mg trans-O-(4-Bromphenylacetyl)-4-(4-dimethylaminomethyl-phenyl)-cyclohexanol

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel VI

Creme für die topische Verabreichung mit 1 g trans-O-(4-Chlorphenylacetyl)-4-(4-dimethylaminomethyl-phenyl)-cyclohexanol

Eine Formulierung für die topische Verabreichung der Verbindungen der Formel I kann folgende Zusammensetzung aufweisen

| | |
|---|---|
| 1. Wirkstoff | 1,0 g |
| 2. Stearylalkohol | 4,0 g |
| 3. Cetylalkohol | 4,0 g |
| 4. Mineralöl | 3,0 g |
| 5. Polysorbat 60 | 4,5 g |
| 6. Sorbitanstearat | 4,5 g |
| 7. Propylenglycol | 10,0 g |
| 8. Methylparaben | 0,18 g |
| 9. Propylparaben | 0,02 g |
| 10. Wasser q. s. | ad 100,00 g |

Die Bestandteile 2-6 werden auf 80°C erwärmt bis alles geschmolzen ist. Danach wird Bestandteil 1 in der öligen Phase gelöst. Bestandteil 7 und 10 werden auf 90°C erwärmt und die Bestandteile 8 und 9 werden in der so erhaltenen wässrigen Phase gelöst. Danach wird die wässrige Phase zur Ölphase gegeben und rasch gerührt, so daß eine Emulsion erhalten wird. Danach läßt man langsam auf 50°C abkühlen um die Emulsion zu verfestigen. Unter weiterem Rühren wird das Präparat auf Raumtemperatur abgekühlt.

Das folgende Beispiel beschreibt die Herstellung eines Futtermittels für Legehennen:

Beispiel VII

Futtermittel für Legehennen, enthaltend als Wirkstoff trans-O-(4-Chlorphenylacetyl)-4-(4-dimethylaminomethylphenyl)-cyclohexanol

| | |
|---|---|
| Mais | 633 g/kg |
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-Mineralstoffmischung | 5 g/kg |
| Wirkstoff | 2 g/kg |

Diese Komponenten in den angegebenen Mengen ergeben nach sorgfältigem Mischen 1 kg Futter.

**Patentansprüche**

**1.** O-Acyl-4-phenyl-cyclohexanole der allgemeinen Formel I,

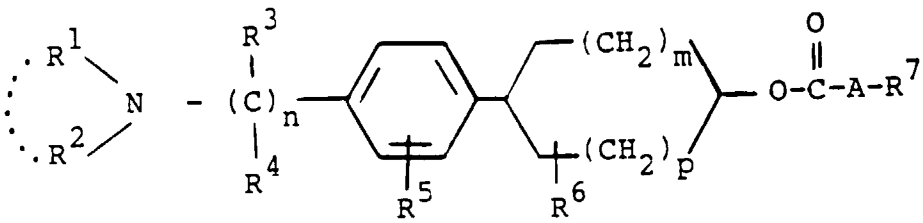

, (I)

in der

n, m und p jeweils die Zahl 1,

$R^1$ und $R^2$, die gleich oder verschieden sein können, ein Wasserstoffatom, eine geradkettige oder verzweigte Alkylgruppe mit 1 bis 6 Kohlenstoffatomen, eine geradkettige oder verzweigte Alkenyl- oder Alkinylgruppe mit jeweils 3 bis 6 Kohlenstoffatomen, wobei deren Doppel- und Dreifachbindungen von der Stickstoff-Kohlenstoff-Bindung isoliert sind und wobei die vorstehend erwähnte Alkyl-, Alkenyl- und Alkinylgruppe durch eine Amino-, Hydroxy-, Alkoxy-, Alkylcarbonyloxy-, Alkylcarbonylamino-, Carboxyl-, Alkoxycarbonyl-, Aminocarbonyl- oder Cyanogruppe substituiert sein kann, wobei die vorstehend erwähnte Amino-, Hydroxy-, Alkoxy-, Alkylcarbonyloxy- und Alkylcarbonylaminogruppe nicht an ein ungesättigtes Kohlenstoffatom und nicht an das Kohlenstoffatom in Position 1 gebunden sein können, oder

$R^1$ und $R^2$ zusammen mit dem dazwischenliegenden Stickstoffatom ein 5- bis 7-gliedriger gesättigter monocyclischer heterocyclischer Ring, wobei in einem so gebildeten 6-gliedrigen gesättigten monocyclischen heterocyclischen Ring eine Methylengruppe in 4-Stellung durch ein Sauerstoff- oder Schwefelatom oder durch eine gegebenenfalls durch eine Alkylgruppe substituierte Iminogruppe ersetzt sein kann,

$R^3$ bis $R^6$, die gleich oder verschieden sein können, jeweils ein Wasserstoffatom oder eine Methylgruppe,

$R^7$ ein Wasserstoffatom, eine Cycloalkylgruppe mit 3 bis 7 Kohlenstoffatomen, eine gegebenenfalls durch ein Fluor-, Chlor- oder Bromatom, durch eine Hydroxy-, Alkyl-, Alkoxy-, Phenylalkoxy-, Phenyl-, Nitro-, Amino-, Alkylamino-, Dialkylamino-, Alkylcarbonylamino-, Cyano-, Carboxy-, Alkoxycarbonyl-, Aminocarbonyl-, Alkylaminocarbonyl-, Dialkylaminocarbonyl-, Trifluormethyl-, Alkylcarbonyloxy-, Aminosulfonyl-, Alkylaminosulfonyl- oder Dialkylaminosulfonylgruppe mono- oder disubstituierte Phenylgruppe, wobei die Substituenten gleich oder verschieden sein können und wobei zwei benachbarte Wasserstoffatome in einer Phenylgruppe durch eine Methylendioxy- oder 1,2-Ethylendioxygruppe ersetzt sein können, eine durch zwei Chlor- oder Bromatome und eine Aminogruppe substituierte Phenylgruppe, eine Naphthyl- oder Tetrahydronaphthylgruppe, eine durch ein Chlor- oder Bromatom oder durch ein oder zwei Alkylgruppen substituierte Thienyl-, Furyl- oder Pyridylgruppe, und

A eine Bindung, eine geradkettige oder verzweigte Alkylengruppe mit 1 bis 10 Kohlenstoffatomen oder eine Alkenylen- oder Alkinylengruppe mit 2 bis 10 Kohlenstoffatomen bedeuten,

wobei alle vorstehend erwähnten Alkyl- und Alkoxyteile, sofern nichts anderes erwähnt wurde, 1 bis 3 Kohlenstoffatome enthalten können, deren Enantiomere, Diastereomere und geometrischen Isomere sowie deren physiologisch verträgliche Salze mit anorganischen oder organischen Säuren.

**2.** O-Acyl-4-phenyl-cyclohexanole der allgemeinen Formel I gemäß Anspruch 1, in der

n, m und p jeweils die Zahl 1,

$R^1$ ein Wasserstoffatom, eine geradkettige oder verzweigte Alkylgruppe mit 1 bis 4 Kohlenstoffatome, die durch eine Aminocarbonylgruppe oder in 2-, 3- oder 4-Stellung durch eine Hydroxy- oder Alkoxygruppe substituiert sein kann, oder eine 2-Propenylengruppe und

$R^2$ ein Wasserstoffatom, eine Alkylgruppe mit 1 bis 4 Kohlenstoffatomen oder eine 2-Propenylengruppe, oder

$R^1$ und $R^2$ zusammen mit dem dazwischenliegenden Stickstoffatom ein 5- oder 6-gliedriger gesättigter monocyclischer heterocyclischer Ring, wobei in einem so gebildeten 6-gliedrigen gesättigten monocyclischen heterocyclischen Ring eine Methylengruppe in 4-Stellung durch ein Sauerstoffatom oder durch eine gegebenenfalls durch eine Alkylgruppe substituierte Iminogruppe ersetzt sein kann,

$R^3$ bis $R^6$ jeweils ein Wasserstoffatom,

$R^7$ ein Wasserstoffatom, eine Cycloalkylgruppe mit 3 bis 6 Kohlenstoffatomen, eine gegebenenfalls in 4-Stellung durch ein Fluor-, Chlor- oder Bromatom, durch eine Alkyl-, Alkoxy-, Phenyl-, Nitro-, oder Trifluormethylgruppe monosubstituierte Phenylgruppe, eine durch zwei Chloratome, ein Chloratom und eine Alkyl- oder Aminogruppe oder durch zwei Alkoxygruppen disubstituierte Phenylgruppe, eine durch zwei Chloratome und eine Aminogruppe trisubstituierte Phenylgruppe, eine 3,4-Methylendioxyphenylgruppe, eine Naphthyl- oder Tetrahydronaphthylgruppe, eine 2-Furylgruppe oder eine gegebenenfalls in 5-Stellung durch ein Chloratom substituierte 2-Thienylgruppe oder eine 3-Pyridylgruppe,

A eine Bindung, eine geradkettige oder verzweigte Alkylengruppe mit 1 bis 6 Kohlenstoffatomen oder eine Alkenylengruppe mit 2 bis 5 Kohlenstoffatomen bedeuten,

wobei alle vorstehend erwähnten Alkyl- und Alkoxyteile, sofern nichts anderes erwähnt wurde, 1 bis 3 Kohlenstoffatome enthalten können, deren Enantiomere, Diastereomere und geometrischen Isomere sowie deren physiologisch verträgliche Salze mit anorganischen oder organischen Säuren.

**3.** O-Acyl-4-phenyl-cyclohexanole der allgemeinen Formel I gemäß Anspruch 1, in der

n, m und p jeweils die Zahl 1,

$R^1$ ein Wasserstoffatom, eine geradkettige oder verzweigte Alkylgruppe mit 1 bis 4 Kohlenstoffatome, die durch eine Aminocarbonylgruppe oder in 2-, 3- oder 4-Stellung durch eine Hydroxy- oder Alkoxygruppe substituiert sein kann, oder eine 2-Propenylengruppe,

$R^2$ ein Wasserstoffatom oder eine Alkylgruppe mit 1 bis 4 Kohlenstoffatomen,

$R^3$ bis $R^6$ jeweils ein Wasserstoffatom,

$R^7$ eine gegebenenfalls in 4-Stellung durch ein Fluor-, Chlor- oder Bromatom, durch eine Methyl-, Trifluormethyl-, Methoxy-, Phenyl- oder Nitrogruppe substituierte Phenylgruppe, eine 3,4-Dichlorphenyl-, 2,4-Dichlorphenyl-, 4-Chlor-3-methylphenyl-, 4-Amino-3-chlorphenyl-, 3,4-Dimethoxyphenyl-, 3,4-Methylendioxyphenyl-, 4-Amino-3,5-dichlorphenyl- oder eine 2-Naphthylgruppe, und

A eine Bindung, eine geradkettige oder verzweigte Alkylengruppe mit 1 bis 5 Kohlenstoffatomen oder eine Alkenylengruppe mit 2 oder 3 Kohlenstoffatomen bedeuten,

wobei alle vorstehend erwähnten Alkyl- und Alkoxyteile, sofern nichts anderes erwähnt wurde, 1 bis 3 Kohlenstoffatome enthalten können, deren Enantiomere, Diastereomere und geometrischen Isomere sowie deren physiologisch verträgliche Salze mit anorganischen oder organischen Säuren.

**4.** Die folgenden Verbindungen der allgemeinen Formel I gemäß Anspruch 1:

(1) trans-O-(p-Tolylacetyl)-4-(4-dimethylaminomethylphenyl)-cyclohexanol und

(2) trans-O-Phenylacetyl-4-(4-dimethylaminomethylphenyl)-cyclohexanol,

und deren physiologisch verträglichen Salze mit anorganischen oder organischen Säuren.

**5.** Arzneimittel, enthaltend eine Verbindung nach mindestens einem der Ansprüche 1 bis 4 neben gegebenenfalls einem oder mehreren inerten Trägerstoffen und/oder Verdünnungsmitteln.

**6.** Verwendung einer Verbindung nach mindestens einem der Ansprüche 1 bis 4 zur Herstellung eines Arzneimittels zur Inhibition der Cholesterolbiosynthese, zur Behandlung oder Prophylaxe von Hyperlipidämien.

**7.** Verwendung einer Verbindung nach mindestens einem der Ansprüche 1 bis 4 zur Herstellung eines Arzneimittels zur Behandlung von Erkrankungen, die mit überhöhter Zellproliferation im Zusammenhang stehen, zur Prophylaxe und Behandlung von Gallensteinleiden oder zur Behandlung von Mykosen.

**8.** Verwendung einer Verbindung nach mindestens einem der Ansprüche 1 bis 4 zur Herstellung eines Futtermittels für Legehennen zur Erzeugung cholesterolarmer Eier.

**9.** Verfahren zur Herstellung eines Arzneimittels gemäß Anspruch 5, dadurch gekennzeichnet, daß auf nichtchemischem Wege eine Verbindung nach mindestens einem der Ansprüche 1 bis 4 in einen oder mehrere inerte Trägerstoffe und/oder Verdünnungsmittel eingearbeitet wird.

**10.** Verfahren zur Herstellung der Verbindungen nach mindestens einem der Ansprüche 1 bis 4, dadurch gekennzeichnet, daß

a) ein 4-Phenylcyclohexanol der allgemeinen Formel II

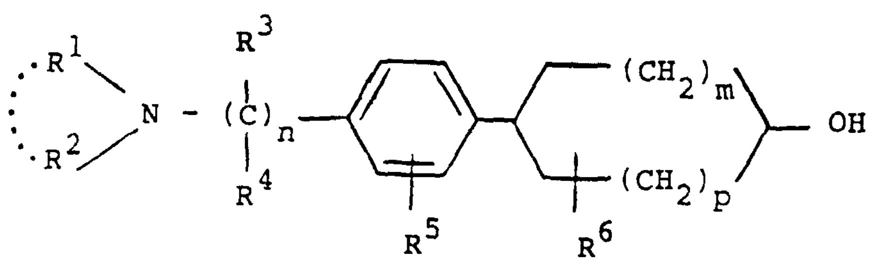

(II)

in der
n, m, p und $R^1$ bis $R^6$ die in den Ansprüchen 1 bis 3 erwähnten Bedeutungen besitzen, mit einer Carbonsäure oder deren reaktiven Derivaten der allgemeinen Formel III

$$R^7 - A - COX, \quad (III)$$

in der
$R^7$ und A die in den Ansprüchen 1 bis 3 erwähnten Bedeutungen besitzen und X eine Hydroxygruppe oder eine reaktive Austrittsgruppe darstellt, bei einer Temperatur zwischen -10 und 150°C umgesetzt wird,

b) ein O-Acyl-4-phenylcycloalkanol der allgemeinen Formel IV

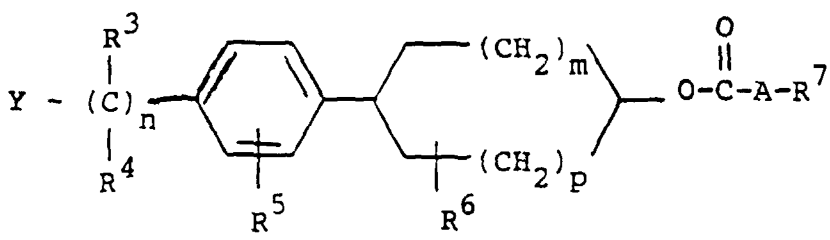

, (IV)

in der
n, m, p, $R^3$ bis $R^7$ und A die in den Ansprüchen 1 bis 3 erwähnten Bedeutungen besitzen, Y eine reaktive Austrittsgruppe darstellt, mit einem Amin der allgemeinen Formel V

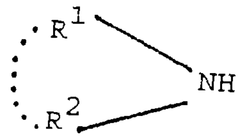

, (V)

in der
$R^1$ und $R^2$ die in den Ansprüchen 1 bis 3 genannten Bedeutungen besitzen, bei einer Temperatur zwischen 0 und 100°C umgesetzt wird, oder

c) zur Herstellung einer Verbindung der allgemeinen Formel I, in der $R^1$ die in den Ansrüchen 1 bis 3 genannten Bedeutungen besitzt und $R^2$ eine geradkettige oder verzweigte Alkylgruppe mit 1 bis 6 Kohlenstoffatomen bedeutet, die auch durch eine Hydroxy-, Alkoxy-, Alkylcarbonyloxy-, Alkylcarbonylamino-, Carboxyl-, Alkoxycarbonyl-, Aminocarbonyl- oder Cyanogruppe substituiert sein kann, wobei die Hydroxy-, Alkoxy-, Alkylcarbonyl-oxy- oder Alkylcarbonylaminogruppe nicht am Kohlenstoffatom in Position 1 gebunden ist, ein O-Acyl-4-phenyl-cycloalkanol der allgemeinen Formel VI

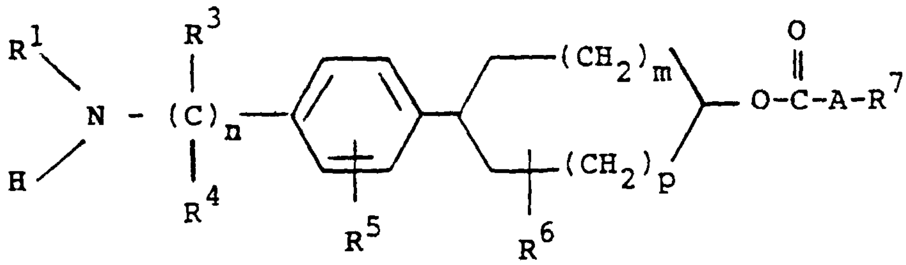

, (VI)

in der
n, m, p, $R^3$ bis $R^7$ und A wie in den Ansprüchen 1 bis 3 definiert sind und $R^1$ die vorstehenden Bedeutungen besitzt, mit einer Verbindung der allgemeinen Formel VII

$$R^{2'} - Z^1 , \qquad (VII)$$

in der
$R^{2'}$ eine geradkettige oder verzweigte Alkylgruppe mit 1 bis 6 Kohlenstoffatomen, die durch eine Hydroxy-, Alkoxy-, Alkylcarbonyloxy-, Alkylcarbonylamino-, Carboxyl-, Alkoxy- carbonyl-, Aminocarbonyl- oder Cyanogruppe substituiert sein kann, wobei die Hydroxy-, Alkoxy-, Alkylcarbonyloxy- oder Alkylcarbonylaminogruppe nicht in Position 1 gebunden ist, darstellt und $Z^1$ eine reaktive Austrittsgruppe bedeutet, bei Temperaturen zwischen 0 und 100°C umgesetzt wird,

und erforderlichenfalls eine gegebenenfalls in den Verbindungen der Formeln II bis VII vorhandene Hydroxy-, Amino-, Alkylamino- oder Carboxylgruppe vor der Durchführung der Reaktionen durch eine Schutzgruppe geschützt wird, wobei nach erfolgter Umsetzung diese Schutzgruppe wieder abgespalten wird, und/oder so erhaltene Verbindungen der allgemeinen Formel I in ihre Salze mit anorganischen oder organischen Säuren übergeführt werden.

## Claims

1. O-Acyl-4-phenyl-cyclohexanols of general formula I

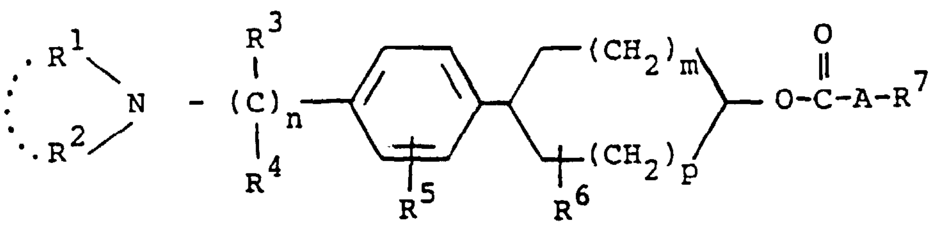

(I)

wherein

n, m and p each denote the number 1,

$R^1$ and $R^2$, which may be identical or different, denote a hydrogen atom, a straight-chained or branched $C_{1-6}$-alkyl group, a straight-chained or branched $C_{3-6}$-alkenyl or alkynyl group, the double and triple bonds thereof being isolated from the nitrogen-carbon bond, whilst the above-mentioned alkyl, alkenyl and alkynyl groups may also be substituted by an amino, hydroxy, alkoxy, alkylcarbonyloxy, alkylcarbonylamino, carboxyl, alkoxycarbonyl, aminocarbonyl or cyano group, and the above-mentioned amino, hydroxy, alkoxy, alkylcarbonyloxy and alkylcarbonylamino groups may not be bound to an unsaturated carbon atom and may not be bound to the carbon atom in position 1, or

$R^1$ and $R^2$ together with the nitrogen atom between them denote a 5- to 7-membered saturated monocyclic heterocyclic ring, whilst in a 6-membered saturated monocyclic heterocyclic ring thus formed a methylene group in the 4-position may be replaced by an oxygen or sulphur atom or by an optionally alkyl-substituted imino group,

$R^3$ to $R^6$, which may be identical or different, each denote a hydrogen atom or a methyl group,

$R^7$ denotes a hydrogen atom, a $C_{3-7}$-cycloalkyl group, a phenyl group optionally mono- or disubstituted by a fluorine, chlorine or bromine atom or by a hydroxy, alkyl, alkoxy, phenylalkoxy, phenyl, nitro, amino, alkylamino, dialkylamino, alkylcarbonylamino, cyano, carboxy, alkoxycarbonyl, aminocarbonyl, alkylaminocarbonyl, dialkylaminocarbonyl, trifluoromethyl, alkylcarbonyloxy, aminosulphonyl, alkylaminosulphonyl or dialkylaminosulphonyl group, wherein the substituents may be identical or different and two adjacent hydrogen atoms in a phenyl group may be replaced by a methylenedioxy or 1,2-ethylenedioxy group, a phenyl group substituted by two chlorine or bromine atoms and an amino group, a naphthyl or tetrahydronaphthyl group, a thienyl, furyl or pyridyl group substituted by a chlorine or bromine atom or by one or two alkyl groups, and

A denotes a bond, a straight-chained or branched $C_{1-10}$-alkylene group or a $C_{2-10}$-alkenylene or alkynylene group,

whilst all the above-mentioned alkyl and alkoxy moieties, unless otherwise specified, may contain 1 to 3 carbon atoms, the enantiomers, diastereomers and geometric isomers thereof and the physiologically acceptable salts thereof with organic or inorganic acids.

**2.** O-Acyl-4-phenyl-cyclohexanols of general formula I according to claim 1, wherein

n, m and p each denote the number 1,

$R^1$ denotes a hydrogen atom, a straight-chained or branched $C_{1-4}$-alkyl group which may be substituted by an aminocarbonyl group or, in the 2-, 3- or 4-position, by a hydroxy or alkoxy group, or a 2-propenylene group, and

$R^2$ denotes a hydrogen atom, a $C_{1-4}$-alkyl group or a 2-propenylene group, or

$R^1$ and $R^2$ together with the nitrogen atom between them denote a 5- or 6-membered saturated monocyclic heterocyclic ring, whilst in a 6-membered saturated monocyclic heterocyclic ring thus formed, a methylene group in the 4-position may be replaced by an oxygen atom or by an optionally alkyl-substituted imino group,

$R^3$ to $R^6$ each denote a hydrogen atom,

$R^7$ denotes a hydrogen atom, a $C_{3-6}$-cycloalkyl group, a phenyl group optionally monosubstituted in the 4-position by a fluorine, chlorine or bromine atom or by an alkyl, alkoxy, phenyl, nitro or trifluoromethyl group, a phenyl group disubstituted by two chlorine atoms, one chlorine atom and an alkyl or amino group or two alkoxy groups, a phenyl group trisubstituted by two chlorine atoms and an amino group, a 3,4-methylenedioxyphenyl group, a naphthyl or tetrahydronaphthyl group, a 2-furyl group or a 2-thienyl group optionally substituted by a chlorine atom in the 5-position or a 3-pyridyl group,

A denotes a bond, a straight-chained or branched $C_{1-6}$-alkylene group or a $C_{2-5}$-alkenylene group,

whilst all the above-mentioned alkyl and alkoxy moieties, unless otherwise specified, may contain 1 to 3 carbon atoms, the enantiomers, diastereomers and geometric isomers thereof and the physiologically acceptable salts thereof with inorganic or organic acids.

**3.** O-Acyl-4-phenyl-cyclohexanols of general formula Ia according to claim 1, wherein

n, m and p each denote the number 1,

$R^1$ denotes a hydrogen atom, a straight-chained or branched $C_{1-4}$-alkyl group which may be substituted by an aminocarbonyl group or, in the 2-, 3- or 4-position, by a hydroxy or alkoxy group, or a 2-propenylene group,

$R^2$ denotes a hydrogen atom or a $C_{1-4}$-alkyl group,

$R^3$ to $R^6$ each denote a hydrogen atom,

$R^7$ denotes a phenyl group optionally substituted in the 4-position by a fluorine, chlorine or bromine atom or by a methyl, trifluoromethyl, methoxy, phenyl or nitro group, a 3,4-dichlorophenyl, 2,4-dichlorophenyl, 4-chloro-3-methylphenyl, 4-amino-3-chlorophenyl, 3,4-dimethoxyphenyl, 3,4-methylenedioxyphenyl, 4-amino-3,5-dichlorophenyl or 2-naphthyl group, and

A denotes a bond, a straight-chained or branched $C_{1-5}$-alkylene group or a $C_{2-3}$-alkenylene group,

wherein all the above-mentioned alkyl and alkoxy moieties, unless otherwise specified, may contain 1 to 3 carbon atoms, the enantiomers, diastereomers and geometric isomers and the physiologically acceptable salts thereof

with inorganic or organic acids.

**4.** The following compounds of general formula I according to claim 1:

(1) trans-O-(p-tolylacetyl)-4-(4-dimethylaminomethylphenyl)-cyclohexanol and

(2) trans-O-(phenylacetyl)-4-(4-dimethylaminomethylphenyl)-cyclohexanol

and the physiologically acceptable salts thereof with inorganic or organic acids.

**5.** Pharmaceutical compositions containing a compound according to at least one of claims 1 to 4 optionally together with one or more inert carriers and/or diluents.

**6.** Use of a compound according to at least one of claims 1 to 4 for preparing a pharmaceutical composition for inhibiting cholesterol biosynthesis, for the treatment or prophylaxis of hyperlipidaemias.

**7.** Use of a compound according to at least one of claims 1 to 4 for preparing a pharmaceutical composition for treating diseases connected with excessive cell proliferation, for the prophylaxis and treatment of gallstone problems or for the treatment of mycoses.

**8.** Use of a compound according to at least one of claims 1 to 4 for preparing a feed for laying hens in order to produce low-cholesterol eggs.

**9.** Process for preparing a pharmaceutical composition according to claim 5, characterised in that a compound according to at least one of claims 1 to 4 is incorporated in one or more inert carriers and/or diluents by a non-chemical method.

**10.** Process for preparing compounds according to at least one of claims 1 to 4, characterised in that

a) a 4-phenylcyclohexanol of general formula II

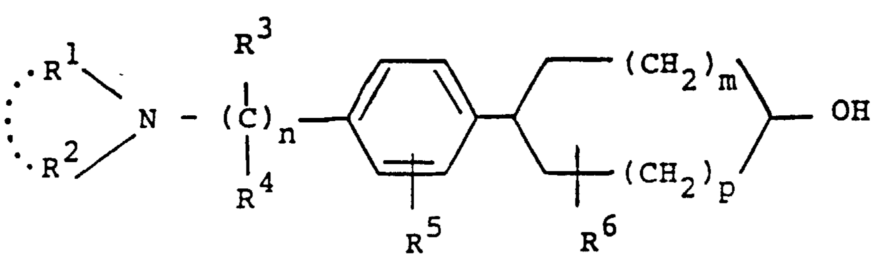

(II)

wherein
n, m, p and $R^1$ to $R^6$ are defined as in claims 1 to 3, is reacted with a carboxylic acid or the reactive derivatives thereof of general formula III

$$R^7 - A - COX \qquad (III)$$

wherein
$R^7$ and A are defined as in claims 1 to 3 and X denotes a hydroxy group or a reactive leaving group, at a temperature between -10 and 150°C,

b) an O-acyl-4-phenylcycloalkanol of general formula IV

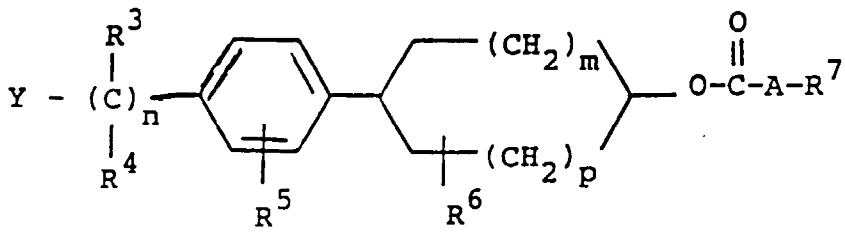

(IV)

wherein
n, m, p, $R^3$ to $R^7$ and A are defined as in claims 1 to 3 and Y denotes a reactive leaving group is reacted with an amine of general formula V

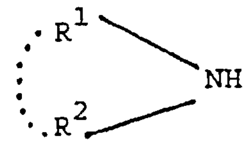

(V)

wherein
$R^1$ and $R^2$ have the meanings given in claims 1 to 3, at a temperature between 0 and 100°C, or

c) in order to prepare a compound of general formula I wherein $R^1$ has the meanings given in claims 1 to 3 and $R^2$ denotes a straight-chained or branched $C_{1-6}$-alkyl group which may also be substituted by a hydroxy, alkoxy, alkylcarbonyloxy, alkylcarbonylamino, carboxyl, alkoxycarbonyl, aminocarbonyl or cyano group, wherein the hydroxy, alkoxy, alkylcarbonyloxy or alkylcarbonylamino group is not bound to the carbon atom in position 1, an O-acyl-4-phenyl-cycloalkanol of general formula VI

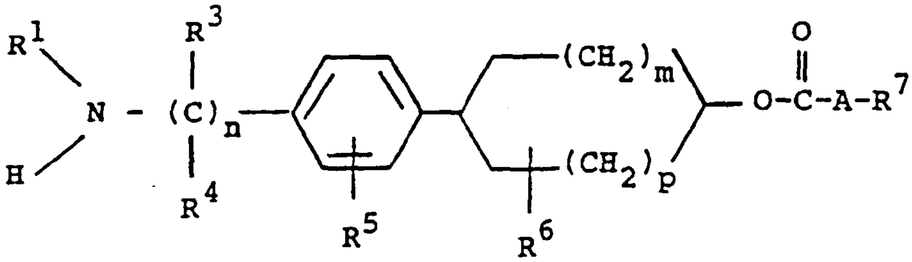

(VI)

wherein
n, m, p, $R^3$ to $R^7$ and A are defined as in claims 1 to 4 and $R^1$ is as hereinbefore defined, is reacted with a compound of general formula VII

$$R^{2'} - Z^1 \quad \text{(VII)}$$

wherein
$R^{2'}$ denotes a straight-chained or branched $C_{1-6}$-alkyl group which may be substituted by a hydroxy, alkoxy, alkylcarbonyloxy, alkylcarbonylamino, carboxyl, alkoxycarbonyl, aminocarbonyl or cyano group, wherein the hydroxy, alkoxy, alkylcarbonyloxy or alkylcarbonylamino group is not bound in position 1, and $Z^1$ denotes a reactive leaving group, at temperatures between 0 and 100°C,
and if necessary any hydroxy, amino, alkylamino or carboxyl group present in the compounds of formulae II to VII is protected before the reactions by a protecting group which is cleaved again after the reaction has taken place, and/or compounds of general formula I thus obtained are converted into the salts thereof with inorganic or organic acids.

## Revendications

1. O-acyl-4-phényl-cyclohexanols de formule générale I

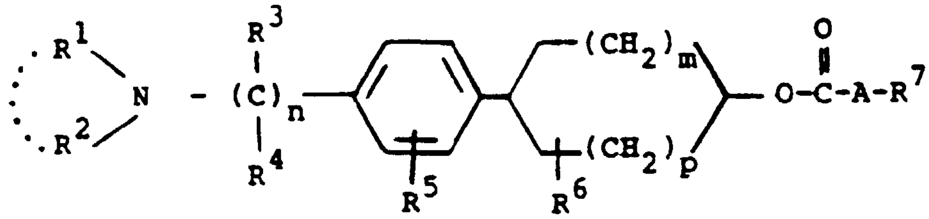

(I)

où

n, m et p représentent chacun le nombre 1,
$R^1$ et $R^2$, qui peuvent être identiques ou différents, représentent un atome d'hydrogène, un groupe alkyle linéaire ou ramifié ayant 1 à 6 atomes de carbone, un groupe alcényle ou alcynyle linéaire ou ramifié ayant chacun 3 à 6 atomes de carbone, dont les doubles et triples liaisons sont isolées de la liaison azote-carbone, et le groupe alkyle, alcényle et alcynyle cité précédemment peut être substitué par un groupe amino, hydroxyle, alcoxy, alkylcarbonyloxy, alkylcarbonylamino, carboxyle, alcoxycarbonyle, aminocarbonyle ou cyano, le groupe amino, hydroxyle, alcoxy, alkylcarbonyloxy et alkylcarbonylamino cité précédemment ne pouvant pas être lié à un atome de carbone insaturé et à l'atome de carbone en position 1, ou
$R^1$ et $R^2$ forment avec l'atome d'azote situé entre eux un cycle hétérocyclique monocyclique saturé à 5 à 7 chaînons, dans un cycle hétérocyclique monocyclique saturé à 6 chaînons ainsi formé un groupe méthylène en position 4 pouvant être remplacé par un atome d'oxygène ou de soufre ou par un groupe imino éventuellement substitué par un groupe alkyle,
$R^3$ à $R^6$, qui peuvent être identiques ou différents, représentent chacun un atome d'hydrogène ou un groupe méthyle,
$R^7$ représente un atome d'hydrogène, un groupe cycloalkyle ayant 3 à 7 atomes de carbone, un groupe phényle éventuellement mono- ou disubstitué par un atome de fluor, de chlore ou de brome, par un groupe hydroxyle, alkyle, alcoxy, phénylalcoxy, phényle, nitro, amino, alkylamino, dialkylamino, alkylcarbonylamino, cyano, carboxyle, alcoxycarbonyle, aminocarbonyle, alkylaminocarbonyle, dialkylaminocarbonyle, trifluorométhyle, alkylcarbonyloxy, aminosulfonyle, alkylaminosulfonyle ou dialkylaminosulfonyle, les substituants pouvant être identiques ou différents et deux atomes d'hydrogène voisins dans un groupe phényle pouvant être remplacés par un groupe méthylènedioxy ou 1,2-éthylènedioxy, un groupe phényle substitué par deux atomes de chlore ou de brome et un groupe amino, un groupe naphtyle ou tétrahydronaphtyle, un groupe thiényle, furyle ou pyridyle substitué par un atome de chlore ou de brome ou par un ou deux groupes alkyle, et
A représente une liaison, un groupe alkylène linéaire ou ramifié ayant 1 à 10 atomes de carbone ou un groupe alcénylène ou alcynylène ayant 2 à 10 atomes de carbone,

où toutes les parties alkyle et alcoxy citées précédemment, sauf indication contraire, peuvent contenir 1 à 3 atomes de carbone, leurs énantiomères, diastéréoisomères et isomères géométriques ainsi que leurs sels physiologiquement acceptables avec des acides inorganiques ou organiques.

2. O-acyl-4-phényl-cyclohexanols de formule générale I selon la revendication 1
où

n, m et p représentent chacun le nombre 1,
$R^1$ représente un atome d'hydrogène, un groupe alkyle linéaire ou ramifié ayant 1 à 4 atomes de carbone, qui peut être substitué par un groupe aminocarbonyle ou en position 2, 3 ou 4 par un groupe hydroxyle ou alcoxy, ou un groupe 2-propénylène et
$R^2$ représente un atome d'hydrogène, un groupe alkyle ayant 1 à 4 atomes de carbone ou un groupe 2-propénylène ou
$R^1$ et $R^2$ forment avec l'atome d'azote situé entre eux un cycle hétérocyclique monocyclique saturé à 5 ou 6 chaînons, où dans un cycle hétérocyclique monocyclique saturé à 6 chaînons ainsi formé un groupe méthylène en position 4 peut être remplacé par un atome d'oxygène ou par un groupe imino éventuellement substitué par un groupe alkyle,

$R^3$ à $R^6$ représentent chacun un atome d'hydrogène,
$R^7$ représente un atome d'hydrogène, un groupe cycloalkyle ayant 3 à 6 atomes de carbone, un groupe phényle éventuellement monosubstitué en position 4 par un atome de fluor, de chlore ou de brome, par un groupe alkyle, alcoxy, phényle, nitro ou trifluorométhyle, un groupe phényle disubstitué par deux atomes de chlore, un atome de chlore et un groupe alkyle ou amino ou par deux groupes alcoxy, un groupe phényle trisubstitué par deux atomes de chlore et un groupe amino, un groupe 3,4-méthylènedioxyphényle, un groupe naphtyle ou tétrahydronaphtyle, un groupe 2-furyle ou un groupe 2-thiényle éventuellement substitué en position 5 par un atome de chlore ou un groupe 3-pyridyle,
A représente une liaison, un groupe alkylène linéaire ou ramifié ayant 1 à 6 atomes de carbone ou un groupe alcénylène ayant 2 à 5 atomes de carbone,

où toutes les parties alkyle et alcoxy citées précédemment, sauf indication contraire, peuvent contenir 1 à 3 atomes de carbone, leurs énantiomères, diastéréoisomères et isomères géométriques ainsi que leurs sels physiologiquement acceptables avec des acides inorganiques ou organiques.

**3.** O-acyl-4-phényl-cyclohexanols de formule générale I selon la revendication 1
où

n, m et p représentent chacun le nombre 1,
$R^1$ représente un atome d'hydrogène, un groupe alkyle linéaire ou ramifié ayant 1 à 4 atomes de carbone, qui peut être substitué par un groupe aminocarbonyle ou en position 2, 3 ou 4 par un groupe hydroxyle ou alcoxy, ou un groupe 2-propénylène,
$R^2$ représente un atome d'hydrogène ou un groupe alkyle ayant 1 à 4 atomes de carbone,
$R^3$ à $R^6$ représentent chacun un atome d'hydrogène,
$R^7$ représente un groupe phényle éventuellement substitué en position 4 par un atome de fluor, de chlore ou de brome, par un groupe méthyle, trifluorométhyle, méthoxy, phényle ou nitro, un groupe 3,4-dichlorophényle, 2,4-dichlorophényle, 4-chloro-3-méthylphényle, 4-amino-3-chlorophényle, 3,4-diméthoxyphényle, 3,4-méthylènedioxyphényle, 4-amino-3,5-dichloro-phényle ou 2-naphtyle, et
A représente une liaison, un groupe alkylène linéaire ou ramifié ayant 1 à 5 atomes de carbone ou un groupe alcénylène ayant 2 ou 3 atomes de carbone,

où toutes les parties alkyle et alcoxy citées précédemment, sauf indication contraire, peuvent contenir 1 à 3 atomes de carbone, leurs énantiomères, diastéréoisomères et isomères géométriques ainsi que leurs sels physiologiquement acceptables avec des acides inorganiques ou organiques.

**4.** Composés suivants de formule générale I selon la revendication 1:

(1) trans-O-(p-tolylacétyl)-4-(4-diméthylaminométhylphényl)-cyclohexanol et
(2) trans-O-phénylacétyl-4-(4-diméthylaminométhylphényl)-cyclohexanol,

et leurs sels physiologiquement acceptables avec des acides inorganiques ou organiques.

**5.** Médicament contenant un composé selon au moins l'une des revendications 1 à 4 outre éventuellement un ou plusieurs supports et/ou diluants inertes.

**6.** Utilisation d'un composé selon au moins l'une des revendications 1 à 4 pour la préparation d'un médicament pour l'inhibition de la biosynthèse du cholestérol, pour le traitement ou la prophylaxie des hyperlipémies.

**7.** Utilisation d'un composé selon au moins l'une des revendications 1 à 4 pour la préparation d'un médicament pour le traitement des maladies qui sont liées à une prolifération cellulaire excessive, pour la prophylaxie et le traitement des affections de type lithiase biliaire ou pour le traitement des mycoses.

**8.** Utilisation d'un composé selon au moins l'une des revendications 1 à 4 pour la préparation d'un aliment pour poules pondeuses pour la production d'oeufs pauvres en cholestérol.

**9.** Procédé de préparation d'un médicament selon la revendication 5 caractérisé en ce que, par voie non chimique, un composé selon au moins l'une des revendications 1 à 4 est incorporé dans un ou plusieurs supports et/ou diluants inertes.

**10.** Procédé de préparation des composés selon au moins l'une des revendications 1 à 4 caractérisé en ce que

a) un 4-phénylcyclohexanol de formule générale II

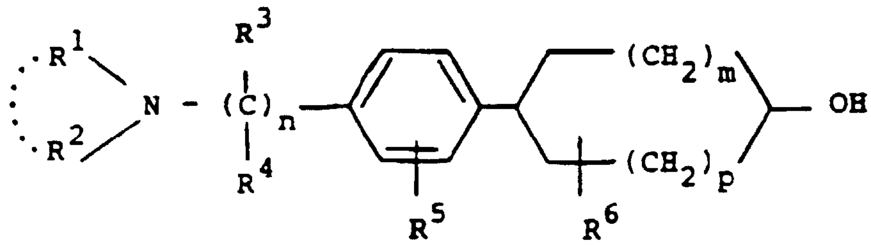

(II)

où
n, m, p et $R^1$ à $R^6$ possèdent les significations citées dans les revendications 1 à 3 est mis à réagir avec un acide carboxylique ou ses dérivés réactifs de formule générale III

$$R^7 - A - COX \quad \text{(III)}$$

où
$R^7$ et A possèdent les significations citées dans les revendications 1 à 3 et X représente un groupe hydroxyle ou un groupe partant réactif, à une température située entre -10 et 150°C,
b) un O-acyl-4-phénylcycloalcanol de formule générale IV

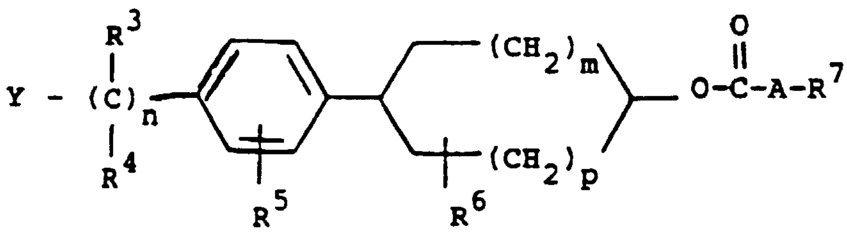

(IV)

où
n, m, p, $R^3$ à $R^7$ et A possèdent les significations citées dans les revendications 1 à 3, Y représente un groupe partant réactif est mis à réagir avec une amine de formule générale V

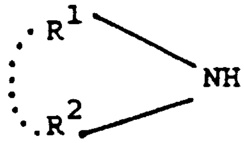

(V)

où
$R^1$ et $R^2$ possèdent les significations citées dans les revendications 1 à 3, à une température située entre 0 et 100°C, ou
c) pour la préparation d'un composé de formule générale I où $R^1$ possède les significations citées dans les revendications 1 à 3 et $R^2$ représente un groupe alkyle linéaire ou ramifié ayant 1 à 6 atomes de carbone, qui peut aussi être substitué par un groupe hydroxyle, alcoxy, alkylcarbonyloxy, alkylcarbonylamino, carboxyle, alcoxycarbonyle, aminocarbonyle ou cyano, où le groupe hydroxyle, alcoxy, alkylcarbonyloxy ou alkylcarbonylamino n'est pas lié à l'atome de carbone en position 1, un O-acyl-4-phényl-cycloalcanol de formule générale VI

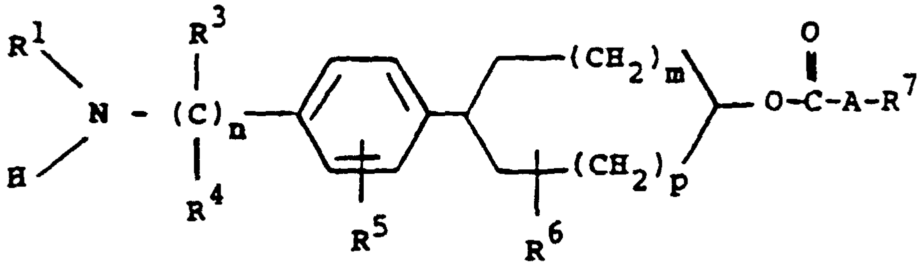

(VI)

où

n, m, p, $R^3$ à $R^7$ et A sont définis comme dans les revendications 1 à 3 et $R^1$ possède les significations précédentes, est mis à réagir avec un composé de formule générale VII

$$R^{2'} - Z^1 \qquad (VII)$$

où

$R^{2'}$ représente un groupe alkyle linéaire ou ramifié de 1 à 6 atomes de carbone qui peut être substitué par un groupe hydroxyle, alcoxy, alkylcarbonyloxy, alkylcarbonylamino, carboxyle, alcoxycarbonyle, aminocarbonyle ou cyano, le groupe hydroxyle, alcoxy, alkylcarbonyloxy ou alkylcarbonylamino n'étant pas lié en position 1, et $Z^1$ représente un groupe partant réactif, à des températures situées entre 0 et 100°C,

et, si nécessaire, un groupe hydroxyle, amino, alkylamino ou carboxyle éventuellement présent dans les composés des formules II à VII est protégé par un groupe protecteur avant la conduite des réactions, ce groupe protecteur étant clivé après la réaction, et/ou les composés de formule générale I ainsi obtenus sont convertis en leurs sels avec des acides inorganiques ou organiques.